# EUROPEAN PATENT SPECIFICATION

(11) **EP 3 544 624 B1**
(45) Date of publication and mention of the grant of the patent: **12.02.2025**
(21) Application number: 17817018.9
(22) Date of filing: 17.11.2017
(51) Int. Cl.: A61K 38/29, A61P 9/04, A61K 31/522, A61K 35/28, A61P 9/00

(54) **TREATMENTS FOR HEART FAILURE AND CARDIAC ISCHAEMIC REPERFUSION INJURY**
BEHANDLUNGEN VON HERZVERSAGEN UND HERZISCHÄMIE/REPERFUSIONSSCHÄDEN
TRAITEMENTS POUR INSUFFISANCE CARDIAQUE ET LÉSION ISCHÉMIQUE/ DE REPERFUSION CARDIAQUE

(30) Priority: 24.11.2016 GB 201619861
(43) Date of publication of application: 02.10.2019
(73) Proprietor: Narodden, Salomon, London, Greater London SW6 3EH (GB)
(72) Inventor: Narodden, Salomon, London, Greater London SW6 3EH (GB)
(74) Representative: Dehns
(86) International application number: PCT/GB2017/053473
(87) International publication number: WO 2018/096319

(56) References cited:
- US-A1- 2009 297 470
- DATABASE MEDLINE [online] US NATIONAL LIBRARY OF MEDICINE (NLM), BETHESDA, MD, US; August 2010 (2010-08-01), ITO YOSHITAKA ET AL: "Cardiac shock wave therapy ameliorates left ventricular remodeling after myocardial ischemia-reperfusion injury in pigs in vivo.", Database accession no. NLM20617568
- ITO YOSHITAKA ET AL: "Cardiac shock wave therapy ameliorates left ventricular remodeling after myocardial ischemia-reperfusion injury in pigs in vivo.", CORONARY ARTERY DISEASE AUG 2010, vol. 21, no. 5, August 2010 (2010-08-01), pages 304 - 311, XP009503304, ISSN: 1473-5830, DOI: 10.1097/MCA.0b013e32833aec62
- DATABASE MEDLINE [online] US NATIONAL LIBRARY OF MEDICINE (NLM), BETHESDA, MD, US; 2010, VASIUK IU A ET AL: "[Efficacy of shock-wave therapy in the treatment of chronic ischemic heart failure].", XP002777909, Database accession no. NLM21591388
- VASIUK IU A ET AL: "[Efficacy of shock-wave therapy in the treatment of chronic ischemic heart failure].", KARDIOLOGIIA 2010, vol. 50, no. 12, 2010, pages 22 - 26, ISSN: 0022-9040
- DATABASE BIOSIS [online] BIOSCIENCES INFORMATION SERVICE, PHILADELPHIA, PA, US; October 2008 (2008-10-01), ITO YOSHITAKA ET AL: "Extracorporeal Cardiac Shock Wave Therapy Ameliorates Left Ventricular Remodeling after Myocardial Ischemia-Reperfusion Injury in Pigs", XP002777908, Database accession no. PREV200900196733
- CIRCULATION, vol. 118, no. 18, Suppl. 2, October 2008 (2008-10-01), 81ST ANNUAL SCIENTIFIC SESSION OF THE AMERICAN-HEART-ASSOCIATION; NEW ORLEANS, LA, USA; NOVEMBER 08 -12, 2008, pages S291, ISSN: 0009-7322
- ITO YOSHITAKA ET AL: "Cardiac shock wave therapy ameliorates left ventricular remodeling after myocardial ischemia-reperfusion injury in pigs in vivo.", CORONARY ARTERY DISEASE AUG 2010, vol. 21, no. 5, August 2010 (2010-08-01), pages 304 - 311, XP009503304, ISSN: 1473-5830
- DATABASE BIOSIS [online] BIOSCIENCES INFORMATION SERVICE, PHILADELPHIA, PA, US; October 2008 (2008-10-01), ITO YOSHITAKA ET AL: "Extracorporeal Cardiac Shock Wave Therapy Ameliorates Left Ventricular Remodeling after Myocardial Ischemia-Reperfusion Injury in Pigs", XP002777908, Database accession no. PREV200900196733
- CIRCULATION, vol. 118, no. 18, Suppl. 2, October 2008 (2008-10-01), 81ST ANNUAL SCIENTIFIC SESSION OF THE AMERICAN-HEART-ASSOCIATION; NEW ORLEANS, LA, USA; NOVEMBER 08 -12, 2008, pages S291, ISSN: 0009-7322
- DATABASE MEDLINE [online] US NATIONAL LIBRARY OF MEDICINE (NLM), BETHESDA, MD, US; 2010, VASIUK IU A ET AL: "[Efficacy of shock-wave therapy in the treatment of chronic ischemic heart failure].", XP002777909, Database accession no. NLM21591388
- KARDIOLOGIIA 2010, vol. 50, no. 12, 2010, pages 22 - 26, ISSN: 0022-9040
- HIROAKI SHIMOKAWA ET AL: "Extracorporeal cardiac shock wave therapy for ischemic heart disease", SHOCK WAVES ; AN INTERNATIONAL JOURNAL ON SHOCK WAVES, DETONATIONS AND EXPLOSIONS - PUBLISHED UNDER THE AUSPICES OF THE INTERNATIONAL SHOCK WAVE INSTIUTE, SPRINGER, BERLIN, DE, vol. 17, no. 6, 29 February 2008 (2008-02-29), pages 449 - 455, XP019620320, ISSN: 1432-2153
- J. VAINER ET AL: "Cardiac shockwave therapy in patients with chronic refractory angina pectoris", NETHERLANDS HEART JOURNAL, vol. 24, no. 5, 2 March 2016 (2016-03-02), NL, pages 343 - 349, XP055447892, ISSN: 1568-5888, DOI: 10.1007/s12471-016-0821-y
- CHINDA KROEKKIAT ET AL: "DIPEPTIDYL PEPTIDASE-4 INHIBITOR ATTENUATED ISCHEMIA-REPERFUSION INJURY VIA PREVENTING MITOCHONDRIAL DYSFUNCTION AND REDUCING CELLULAR APOPTOSIS", JOURNAL OF THE AMERICAN COLLEGE OF CARDIOLOGY, vol. 61, no. 10, 12 March 2013 (2013-03-12), XP028753825, ISSN: 0735-1097, DOI: 10.1016/S0735-1097(13)60221-1
- MADOKA IHARA ET AL: "An interaction between glucagon-like peptide-1 and adenosine contributes to cardioprotection of a dipeptidyl peptidase 4 inhibitor from myocardial ischemia-reperfusion injury", AMERICAN JOURNAL OF PHYSIOLOGY: HEART AND CIRCULATORY PHYSIOLOGY, vol. 308, no. 10, 15 May 2015 (2015-05-15), US, pages H1287 - H1297, XP055447904, ISSN: 0363-6135, DOI: 10.1152/ajpheart.00835.2014
- AYAKO TAKAHASHI ET AL: "Dipeptidyl-peptidase IV inhibition improves pathophysiology of heart failure and increases survival rate in pressure-overloaded mice", AMERICAN JOURNAL OF PHYSIOLOGY: HEART AND CIRCULATORY PHYSIOLOGY, vol. 304, no. 10, 15 May 2013 (2013-05-15), US, pages H1361 - H1369, XP055447889, ISSN: 0363-6135, DOI: 10.1152/ajpheart.00454.2012

## Description

The present invention relates to the use of extracorporeal cardiac shockwave therapy in the treatment or prophylaxis of heart disease, particularly heart failure and cardiac ischaemic reperfusion injury. In particular, the invention relates to agents, particularly to inhibitors of dipeptidyl peptidase-4 (DPP-4 inhibitors) and pharmaceutical compositions comprising said agents, and their use in combination with shockwave therapy for the treatment or prophylaxis of heart disorders, such as heart failure and cardiac ischaemic reperfusion injury. The agents may be useful alone or in combination with other therapeutically active agents, such as stem cell mobilizing agents, particularly parathyroid hormone, and stem cells, such as bone marrow-derived mononuclear cells, particularly hematopoietic progenitor cells.

Heart failure affects 1-2% of the population in the UK and it is increasingly prevalent with age (1% under 65, 6-7% of those aged 75-84, 12-22% over 85 years old). The major cause of heart failure in the UK is due to coronary artery disease (about 70% of heart failure patients) with an average life expectancy of 3 years after diagnosis, which is worse than many forms of malignancy and about 14% of heart failure patients die within 6 months of diagnosis.

Heart failure accounts for 5% of medical admissions and 1-2% of the NHS budget. The syndrome not only carries a significant health and economic burden but also has adverse effects on the quality of life. As the population ages over the next 25 years, it is estimated that there will be an increase of 50% in hospital admissions with heart failure.

Coronary heart disease commonly culminates in a myocardial infarction (heart attack) and whilst primary angioplasties and stents to treat patients with heart attacks have increased the chance of their survival, the prevalence of heart failure is actually increasing. It is thought that this increase in the prevalence of heart failure is partly attributable to the fact that reperfusion injury accounts for 50% of the final infarct size and 25% of acute heart failure cases.

Despite the advancement of medical therapies and electrophysiological interventions, many patients with congestive (chronic) heart failure remain symptomatic with reduced quality of life and an unacceptably poor prognosis. A radical treatment such as cardiac transplantation or the implant of a circulatory assisted device could be considered in some cases, but there is a lack of organs available and a 10-year survival rate of only about 50% in heart transplant patients.

In subjects suffering from heart attacks, a myocardial infarction results in a severe deficiency in oxygen to cardiac tissue and subsequent treatment (e.g. removal or bypass of the blockage) allows reperfusion of the tissue and consequent reoxygenation the cardiac cells. However, when cells are exposed to severe deprivation of oxygen followed by reoxygenation, a proportion of cells in the tissue will exceed a certain stress threshold, which commences an irreversible process of programmed cell death known as apoptosis. Whilst the cells may remain structurally intact, they will be removed from the body in a controlled fashion because they are no long viable. Thus, these "stress" conditions are common in subjects suffering from heart attacks and the cardiomyocytes in heart muscles which exceed the oxygen deprivation/reoxygenation stress threshold are replaced with a scar tissue, so-called cardiac ischaemic reperfusion injury. The loss of viable heart muscle tissue impairs the ability of the heart to pump and, in the extreme circumstances, results in heart failure. As noted above, the prognosis in such patients is poor.

Heart homeostasis is maintained by multipotent cardiac stem cells and this capacity for developmental plasticity holds promise for the prevention and treatment of heart failure in humans by reconstituting infarcted tissue with viable progenitor cells. Cellular therapy indeed can produce functional improvement following myocardial infarction. For instance, stem cells have been administered to failing hearts directly through various surgical methods, including intravenous, intracoronary, transendocardial and intramyocardial injections. However, the improvements in heart function observed to date are minor. This may be due to poor cell retention within the heart, poor cell survival and/or the fact that a single dose might not be sufficient to promote a sustained improvement in left ventricular function. Moreover, there are numerous disadvantages to these invasive procedures, not least the potentially fatal complications associated with invasive heart surgery. Indeed, surgical administration of stem cells is expensive and time consuming, due in part to the process of producing stem cells.

Hence there is a need for a novel therapeutic approach for heart failure. In particular, methods to abrogate the death of cardiomyocytes in heart attack patients could be beneficial to reduce the likelihood of heart failure and improve the prognosis of patents with, or at risk of, heart failure.

Ito et al. (Coronary Artery Disease, 2010, vol. 21(5), pp. 304-311) describes a study on the effects of extracorporeal cardiac shockwave therapy following myocardial I/R injury in pigs.

US 2009/297470 describes the use of stem cell mobilisers, particularly parathyroid hormone and G-CSF, to recruit stem cells into tissue suffering from ischaemia. It indicates that DPP-IV inhibitors may potentiate the effects of such stem cell mobilisers.

In work leading up to the present invention, it was surprisingly determined that it is possible to rescue hypoxic cardiomyocytes from apoptosis by exposing the cells to low energy shockwaves. Notably, the inventor has established that treatment of hypoxic cardiomyocytes with shockwaves increases the phosphorylation, i.e. activation, of Protein kinase B (also known as Akt). Akt is a key regulator of apoptosis and activated Akt phosphorylates and inhibits various pro-apoptotic proteins, including Bcl-2 family members Bad, Bax, caspase-9, GSK-3, and FoxO1. However, application of an inhibitor of the PI3K/Akt pathway (LY294002) did not affect the anti-apoptotic effect caused by the application of shockwaves, i.e. the anti-apoptotic effect of shockwave treatment is independent of the PI3K/Akt pathway (see Example 5). Furthermore, the inventor has demonstrated that expression of SDF1, which is known to be anti-apoptotic, is not increased in cardiomyocytes exposed to shockwaves. Interestingly however, shockwave treatment of whole cardiac tissue does result in an increase of various anti-apoptotic proteins, including SDF1, MCP1, ANG1, VEGF-A, NOS3 and TAC-1. These findings have led the inventor to propose new therapies for the treatment of various heart disorders using shockwave therapies, particularly in the treatment of damage caused by hypoxia followed by normoxia, i.e. cardiac ischaemic reperfusion injuries.

Thus, disclosed herein are methods and agents for reducing or minimising apoptosis (i.e. cell death) of cells in heart tissue (e.g. cardiomyocytes). More specifically, the use of shockwave therapy (i.e. extracorporeal cardiac shockwave therapy) alone or in combination with other therapies, particularly pharmaceutical or pharmacological therapies (referred to herein as combination therapies or combined therapies), to inhibit, prevent or minimise apoptosis of cardiac cells, particularly cardiomyocytes is disclosed herein. Such therapeutic methods and pharmaceutical agents may be combined with cellular therapies to repair and/or restore cardiac tissue and function.

For instance, methods are disclosed herein for treating subjects suffering from acute myocardial infarction by administering extracorporeal cardiac shockwave therapy, particularly as an adjuvant treatment alongside coronary angioplasty and stenting. It is advantageous to commence the extracorporeal cardiac shockwave therapy as soon as possible after a myocardial infarction. However, for patients admitted to hospital following a delay and who do not qualify for acute revascularisation or have contraindications for the procedure, the extracorporeal cardiac shockwave therapy may be used alone. Whilst not wishing to be bound by theory, it is thought that the extracorporeal cardiac shockwave therapy may delay the progressive loss of the remaining cardiomyocytes via the prevention of apoptosis, which may be particularly advantageous in patients with established infarctions, i.e. to prevent, reduce or minimise the probability of heart failure.

As noted above, it has been found that the shockwave therapy is effective as an anti-apoptotic therapy for cardiomyocytes *in vitro,* e.g. at preventing, reducing or minimising apoptosis of cardiomyocytes *in vitro.* Accordingly, disclosed herein are methods and means for preventing, reducing or minimising apoptosis of cardiomyocytes *in vitro* (e.g. in culture, such as in the cultivation of cardiomyocytes for the production of artificial or synthetic tissues) or ex *vivo* (e.g. in heart tissue removed from a subject, e.g. in a heart or heart tissue, e.g. valve, for transplantation).

As mentioned above, the inventor has established that the exposure of heart tissue to shockwaves increases the secretion of various chemokines, particularly pro-survival chemokines such as SDF-1 (stromal cell-derived factor 1, also known as C-X-C motif chemokine 12 (CXCL12), MCP1 (monocyte chemoattractant protein 1, also known as CCL2), substance P and IGF1 (Insulin-like growth factor 1), i.e. shockwaves increase the pro-survival "secretome" in heart tissue. Notably, it is thought that the secretome may be in the form of one or more exosomes, e.g. cell-derived vesicles containing one or more chemokines. Whilst the increase in the pro-survival secretome (or exosomes) does not explain the anti-apoptotic effect of shockwaves on cardiomyocytes, it is thought that the increase in these pro-survival chemokines may enhance the survival of cardiac cells exposed to the stress conditions associated with cardiac ischaemia and reperfusion. Notably however, the rapid turnover of chemokines means that the increased pro-survival secretome caused by shockwave therapy may be transient, meaning that repeated shockwave treatments might be necessary to maximise the effects of shockwave therapy described above. However, in some instances, repeated shockwave therapy may be or become impractical. Generally, it is preferable to avoid the need for repeated and regular visits to the clinic as this creates a burden both for the patient and healthcare professionals. Moreover, repeated shockwave treatments may cause discomfort resulting in missed treatments, which may reduce the overall efficacy of the treatment.

The rapid turnover of chemokines is a result of their degradation and/or inactivation. Various enzymes are involved in the degradation of chemokines *in vivo*, such as dipeptidyl peptidase-IV (DPP-IV or DPP-4), which is also known as adenosine deaminase complexing protein 2 or cluster of differentiation 26 (CD26). DPP-4 is an antigenic enzyme expressed on the surface of most cell types. It is an intrinsic membrane glycoprotein and a serine exopeptidase that cleaves X-proline dipeptides from the N-terminus of polypeptides. Thus, DPP-4 has a diverse range of substrates, including SDF-1, MCP1, substance P and IGF1.

Accordingly, the inventor has determined that shockwave therapy may advantageously be combined with one or more protease inhibitors to reduce, prevent or minimise the degradation and/or inactivation of chemokines induced by shockwave therapy, i.e. to prolong the half-life of the pro-survival secretome (which may be in the form of one or more exosomes) of heart tissue resulting from shockwave therapy (referred to herein as a combination therapy or combined therapy). For instance, DPP-4 inhibitors are well-known in the art and can be combined readily with the shockwave therapy methods and uses described above.

Thus, DPP-4 inhibitors may potentiate the effect of shockwave therapy in the methods and uses described above, i.e. may potentiate or enhance the anti-apoptotic effect of shockwaves on heart tissue, particularly cardiomyocytes, thereby improving the treatment of cardiac ischaemic reperfusion injury or minimising or preventing the damage of heart tissue, particularly heart muscle, caused by cardiac ischaemia and reperfusion, e.g. further improving the prognosis of a subject with, or at risk of, heart failure. Notably, the combined therapy (combination therapy) may function to reduce the size of the final infarct and/or prevent, retard or delay the progression to heart failure.

Accordingly, the invention provides a DPP-4 inhibitor, or a pharmaceutical composition containing said inhibitor, for use in:
(a) treating or preventing heart failure; or
(b) treating cardiac ischaemic reperfusion injury,
in a subject, wherein said subject has been administered extracorporeal cardiac shockwave therapy and wherein said inhibitor or composition is for administration prior to, simultaneously with, and/or after administration of said shockwave therapy.

Thus, disclosed herein is a DPP-4 inhibitor, or a pharmaceutical composition containing said inhibitor, for use in improving the prognosis of a subject with, or at risk of, heart failure, wherein said subject has been administered extracorporeal cardiac shockwave therapy and wherein said inhibitor or composition is for administration prior to, simultaneously with, and/or after administration of said shockwave therapy.

Advantageously, the inventor has also determined that the combination of shockwave treatment and DPP-4 inhibitor therapy also functions to promote angiogenesis in heart tissue, which may be particularly beneficial in the treatment of subjects with heart failure (e.g. chronic ischaemic heart disease or chronic myocardial ischemia) or cardiac ischaemic reperfusion injury.

Thus, disclosed herein is a DPP-4 inhibitor, or a pharmaceutical composition containing said inhibitor, for use in inducing angiogenesis in the heart tissue of a subject (e.g. a subject with heart failure (e.g. chronic ischaemic heart disease or chronic myocardial ischemia) or cardiac ischaemic reperfusion injury), wherein said subject has been administered extracorporeal cardiac shockwave therapy and wherein said inhibitor or composition is for administration prior to, simultaneously with, and/or after administration of said shockwave therapy. Thus, the invention provides a DPP-4 inhibitor, or a pharmaceutical composition containing said inhibitor, for use in treating or preventing heart failure (e.g. chronic ischaemic heart disease or chronic myocardial ischemia) or treating cardiac ischaemic reperfusion injury in a subject by inducing angiogenesis in the heart tissue of said subject, wherein said subject has been administered extracorporeal cardiac shockwave therapy and wherein said inhibitor or composition is for administration prior to, simultaneously with, and/or after administration of said shockwave therapy.

It will be evident that the uses described above for inducing angiogenesis in the heart tissue of a subject may also be used for improving the prognosis of a subject with, or at risk of, heart failure.

Stem cells are also known to secrete paracrine factors that have anti-apoptotic effects that could be beneficial in patients suffering from heart attacks and/or heart failure, particularly heart failure caused by ischaemic reperfusion injury. However, as discussed above, the administration of stem cells to the heart is very invasive, expensive and associated with poor cell retention. Moreover, whilst the shockwave therapies described above (including the combined therapy) may minimise the damage to heart tissue caused by a myocardial infarction, e.g. cardiac ischaemic reperfusion injury, they do not provide any functional benefit to the heart in terms of the improvement of the ejection fraction, i.e. the shockwave therapies described above do not restore the function of the heart or repair damage caused by a myocardial infarction, e.g. cardiac ischaemic reperfusion injury.

Thus, the inventor has further determined that the uses described above may be further enhanced by providing subjects with stem cells or progenitor cells that can replace cardiac cells, particularly cardiomyocytes, that died as a consequence of the stress conditions associated with myocardial infarction, e.g. ischaemic reperfusion injury. In this respect, it is thought that the pro-survival secretome (which may be in the form of one or more exosomes) induced in heart tissue on exposure to shockwaves may function to attract or recruit stem cells to the damaged tissue, thereby enhancing the retention of the cells within the heart and enabling the effective repair of damaged tissue (i.e. restoring, at least some of, the lost cardiac function). In other words, the shockwave therapy may be seen to provide an environment that facilitates or potentiates the retention of stem cells within heart tissue and subsequent engraftment of said cells within the heart tissue. Whilst the stem cells may be administered to the heart tissue directly, e.g. via intravenous, intracoronary, transendocardial and/or intramyocardial injection as described above, it will be evident from the discussion below that the present invention removes the need for invasive administration of stem cells.

In this respect, in addition to providing an environment that facilitates or potentiates the retention of stem cells within heart tissue, the heart tissue secretome (which may be in the form of one or more exosomes) induced by the shockwave therapy may function to attract or recruit stem cells from sites other than the heart, e.g. stem cells that are present in blood. In other words, the therapy disclosed herein may facilitate the homing of stem cells to the heart. This may enable stem cells to be administered non-invasively, i.e. by injection at a site other than the heart, e.g. injection into the peripheral blood circulation. Thus, the present invention obviates the need for open heart surgery and direct injection of stem cells to heart tissue, e.g. using catheters into the coronary blood supply, is not necessary.

Advantageously, the present invention supports the use of endogenous stem cells, e.g. stem cells that are present in the blood of the subject to be treated. Thus, the shockwave therapy may result in the non-invasive homing (i.e. recruitment and subsequent engraftment) of stem cells in the peripheral circulation to the heart tissue. In this respect, it may be useful to increase the number of stem cells present in the blood, e.g. using a pharmacological stem cell mobiliser, such as parathyroid hormone as discussed in more detail below.

Thus, in some embodiments, the DPP-4 inhibitor or pharmaceutical composition comprising said inhibitor is provided as a combined preparation with a pharmacological stem cell mobiliser, e.g. parathyroid hormone or a fragment thereof, for separate, simultaneous or sequential use or administration to the subject (a so-called combination therapy).

In some subjects (i.e. patients), it may be beneficial to harvest stem cells from the subject (e.g. prior to the start of treatment) and administer said cells to the subject (e.g. via injection into the peripheral circulation) at the most appropriate time, e.g. prior to shockwave therapy as discussed in more detail below. For instance, it may be necessary to harvest or obtain stem cells from a subject if the subject is unresponsive to agents used to mobilise the stem cells, e.g. parathyroid hormone, or if such agents are contraindicated. It may be useful to expand the population of stem cells *in vitro* (i.e. to culture the subject's endogenous stem cells *in vitro*) prior to administration. Such expansion methods are well-known in the art.

Thus, in some embodiments, the DPP-4 inhibitor or pharmaceutical composition comprising said inhibitor is provided as a combined preparation with stem cells for separate, simultaneous or sequential use or administration to a subject (a so-called combination therapy).

Thus, in some embodiments, the stem cells are obtained from said subject and optionally expanded *in vitro* prior to administration. Thus, in some embodiments, the stem cells are exogenous stem cells, e.g. autogeneic stem cells.

In still further embodiments, stem cells may be used in combination with pharmacological agents suitable for mobilising stem cells, such as parathyroid hormone or a fragment thereof (a so-called combination therapy).

In some instances, it may be desirable or necessary to use stem cells from a donor subject. Thus, in some embodiments, the stem cells are exogenous donor stem cells, i.e. allogeneic stem cells.

It will be evident that the stem cells obtained or harvested from a subject may not be suitable for immediate administration, i.e. it may be necessary or advantageous to modify the stem cells prior to administration. For instance, as mentioned above, it may be useful to expand or cultivate the stem cells *in vitro* to increase the number of stem cells for administration. Additionally or alternatively, it may be useful to isolate or separate different stem cell types prior to administration. Furthermore, in some embodiments the stem cells may be treated to differentiate the cells to make them more suitable for use, e.g. to differentiate the cells into cell types that may be particularly advantageous in the therapies of the invention, e.g. M2c macrophage cells as discussed below. For instance, harvesting or obtaining stem cells may involve isolating blood progenitor cells from a subject (e.g. the subject to be treated or a donor subject), such as bone marrow-derived mononuclear cells, and cultivating said cells *in vitro* under conditions suitable to the preferred stem cells or populations of stem cells described in more detail below. However, in some embodiments, the blood progenitor cells do not undergo differentiation *in vitro,* i.e. in some embodiments the stem cells for administration are blood progenitor cells, e.g. bone marrow-derived mononuclear cells.

As discussed in more detail in the Examples below, the inventor has determined that exposure of vascular endothelial cells to shockwaves results in the rapid and transient increase in the gene expression of SDF-1. Maximum expression of SDF-1 is seen between 2-6 hours, i.e. about 4 hours, after exposure to shockwaves, returning to baseline levels by the 24 hour time point. A similar expression pattern was observed for VEGFA and MCP1 in cardiac fibroblasts, in which maximum expression is observed around 3 hours after exposure to shockwaves. However, while exposure to shockwaves increases SDF-1 expression in fibroblasts, there is a lag in expression such that maximum expression is not seen until at least about 24 hours after exposure to shockwaves.

As discussed above, it is thought that the secretome (which may be in the form of one or more exosomes) induced by shockwave therapy may act to recruit stem cells to cardiac tissue and potentiate their retention in said tissue. Accordingly, in some embodiments it may be advantageous to administer stem cells and/or a pharmacological mobiliser of stem cells prior to the shockwave therapy. For instance, stem cells and/or a pharmacological mobiliser of stem cells may be administered (i.e. for administration) at least 8 hours before the shockwave therapy, e.g. at least 12, 16, 20, 24 hours before the shockwave therapy. In some embodiments, stem cells and/or a pharmacological mobiliser of stem cells may be administered (i.e. for administration) at least 24, 30, 36, 42 or 48 hours before the shockwave therapy.

In some embodiments it may be useful to administer stem cells and/or a pharmacological mobiliser of stem cells immediately after shockwave therapy, i.e. as soon as possible after shockwave therapy. This may be in addition to the prior administration described above or as an alternative embodiment, i.e. in some embodiments the stem cells and/or a pharmacological mobiliser of stem cells are not administered before shockwave therapy. Administration immediately or as soon as possible after shockwave therapy means within minutes or hours after shockwave therapy has been completed, e.g. within 5, 10, 15, 20, 30, 45, 60, 90 or 120 minutes of the completion of shockwave therapy, i.e. the completion of a shockwave therapy dose, e.g. 500-2000 pulses. For instance, administration may be within 1, 2, 3, 4, 5, 6, 7, 8, 9, 10, 11 or 12 hours of the completion of shockwave therapy. As discussed further below, in some embodiments, the shockwave therapy dose (e.g. 500-2000 pulses) may be administered over more than one session, i.e. administration of the dose may be interrupted. Thus, in some embodiments, the stem cells and/or a pharmacological mobiliser of stem cells may be administered during the shockwave therapy, e.g. during an interruption in the administration of a shockwave dose.

The terms "shockwave therapy" and "Extracorporeal Shockwave Therapy" (ESWT) are used interchangeably herein and refer to the administration of high amplitude pulses of mechanical energy (acoustic pressure waves), similar to soundwaves, typically generated by an electromagnetic coil. In particular, the ESWT refers to the use of "low energy" shockwaves. In this respect, "high energy" shockwaves may be defined as those used in "Extracorporeal shock wave lithotripsy" (ESWL), which is a non-invasive treatment of kidney stones. Thus, the "low energy" shockwaves for use in the present invention are distinct from shockwaves used in ESWL.

Thus, low energy shockwaves may be defined as shockwaves having energy of about 0.01-0.50 mJ/mm² (approximately 0.1-5 Bar). In contrast, high energy shockwave may be defined as shockwaves having energy of at least 0.85 mJ/mm² (approximately 8.5 Bar). Thus, in a preferred embodiment, the low energy shockwaves for use in the present invention have energy of approximately 0.01-0.45 mJ/mm², e.g. 0.02-0.40, 0.03-0.35, 0.04-0.30 or 0.05-0.25 mJ/mm² (approximately 0.1-4.5 Bar, e.g. 0.2-4, 0.3-3.5, 0.4-3.0 or 0.5-2.5 Bar). For instance, the low energy shockwaves for use in the present invention have energy of about 0.05, 0.10, 0.15, 0.2 or 0.25 mJ/mm² (approximately 0.5, 1, 1.5, 2 or 2.5 Bar).

The low energy shockwaves may be administered using any suitable means known in the art. For instance, the shockwave therapy may be administered using an electro-hydraulic extracorporeal shockwave lithotripter, such as a DUOLITH^{®} SD1 lithotripter.

The Examples describe the administration of shockwaves *in vitro* using apparatus that generates radial shockwaves. Radial shockwaves spread out over a large surface area and thus do not penetrate deep tissue. As such, radial shockwaves typically find utility in the treatment of near surface pathologies such as myofascial pain syndromes. Accordingly, radial shockwaves are particularly suitable for *in vitro* experiments, where deep penetration is not required. In contrast, the uses of the invention typically use apparatus capable of generating focussed shockwaves, which are capable of penetrating deep tissue, such as the heart. Focussed shockwaves have a small focal point that enables a specific area of tissue to be targeted. Both radial shockwaves and focussed shockwaves are "pressure waves" which generate similar biological responses, i.e. similar responses in cells and tissues. Thus, the primary difference between radial and focussed shockwaves in the context of the present invention relates to the depth of tissue penetration. Accordingly, in a preferred aspect of the invention, the shockwaves used in the uses of the invention are focussed shockwaves.

The term "Extracorporeal" means that the shockwaves are generated externally to the body and transmitted, e.g. from a pad, through the skin.

Shockwave therapy involves the administration of multiple low energy shockwave pulses. For instance, in some embodiments, shockwave therapy (i.e. a dose of low energy shockwaves) comprises administration of at least about 500 shockwave pulses, such as at least 750, 1000, 1100, 1200, 1300, 1400, 1500, 1600, 1700, 1800, 1900 or 2000 shockwave pulses. Thus, in some embodiments, shockwave therapy comprises administration of about 500-2000 pulses, e.g. 600-1900, 700-1800, 800-1700, 900-1600 or 1000-1500 pulses.

The shockwave therapy for use in the present invention is extracorporeal cardiac shockwave therapy, meaning that the shockwaves are focussed on the cardiac tissue, as described in more detail below. In particular, the shockwave pulses are delivered (i.e. administered) during the isovolumic contraction and/or isovolumic relaxation periods of the cardiac cycle. Thus, in some embodiments, shockwave therapy comprises administration of shockwave pulses (i.e. one or more pulses) during the isovolumic contraction and/or isovolumic relaxation period of the heart.

The isovolumic (isovolumetric) contraction period is the time in early systole during which the ventricles contract with no volume change (isovolumetrically). This short-lasting portion of their contraction takes place during a moment when the heart valves are all closed.

The isovolumic relaxation time (IVRT) period refers to an interval in the cardiac cycle from closure of the aortic valve to onset of filling by opening of the mitral valve.

It will be evident that, depending on the heart rate, more than one shockwave pulse may be delivered during each isovolumic contraction and/or isovolumic relaxation period. Accordingly, the length of treatment (i.e. the amount of time required to deliver (administer) as dose of shockwaves, e.g. 500-2000 pulses) will depend on the heart rate of the subject to be treated. Nevertheless, in some embodiments, it is preferred that all of the shockwave pulses (e.g. 500-2000 pulses) are administered within a 4 hour period, preferably within a 3 hour period or 2 hour period, e.g. within 1 hour. In some instances, it may not be possible to administer all of the shockwave pulses (i.e. a dose of shockwave pulses) in a single session, e.g. due to patient discomfort. Thus, in some embodiments, the shockwave pulses are administered in more than one session, i.e. the 500-2000 pulses are administered over multiple sessions. Preferably all of the sessions are within the time period mentioned above, i.e. within a 4 hour period.

The shockwave pulses are delivered (i.e. administered) to the diseased segments or areas of the heart (e.g. the infarcted tissue) and its surrounding cardiac tissue. In some embodiments, the focal point of the shockwave pulses may be targeted to a specific region of the cardiac tissue, i.e. the diseased segments.

In some circumstances, e.g. in the case of an acute situation, such as a subject experiencing a myocardial infarction, there may be insufficient time to determine the precise location of the diseased (infarcted) segments, i.e. to map the diseased segments. Thus, in some embodiments, the location of the diseased or affected (e.g. infarcted) segments may be determined generally using electrocardiogram (ECG) pattern recognition and/or echocardiography (echo) visualisation (i.e. a cardiac echo, which is a sonogram of the heart). Thus, in some embodiments, the region of the heart (i.e. cardiac tissue) to be targeted by shockwave therapy (i.e. the region on which the focal point of the shockwave pulses is targeted) is determined by echocardiography (echo) visualisation and/or ECG pattern recognition.

In some cases, e.g. in the case of a chronic situation (e.g. the treatment of a subject with heart failure) or after the condition of a patient has been stabilised following myocardial infarction, the location of diseased segments of the heart (e.g. infarcted and/or scar tissue) may be determined precisely using cardiac magnetic resonance imaging (MRI). In particular, cardiac MRI may be used to determine the area at risk, salvage index and to measure other functional indices. Cardiac MRI should ideally be performed as soon as possible after the myocardial infarction, e.g. within one week of myocardial infarction.

Thus, in some embodiments, the region of the heart (i.e. cardiac tissue) to be targeted by shockwave therapy (i.e. the region on which the focal point of the shockwave pulses is targeted) is determined by MRI. In other words, a step of determining the size of the diseased segment of the cardiac tissue may be required, i.e. determining or evaluating the area and/or volume of the infarcted tissue, e.g. scar tissue.

Cardiac MRI may be used to assess the efficacy of the therapeutic uses of the invention. Thus, a step of MRI the heart of a subject following treatment according to the invention may be used to determine the efficacy of the treatment and/or to determine the prognosis of the subject. Such steps may be performed at appropriate time intervals, e.g. daily, weekly, monthly and/or annually.

As discussed in more detail below, heart failure is a progressive disease. Thus, some subjects (e.g. subjects with chronic heart failure) may benefit from repeated shockwave treatment sessions in combination with the pharmacological treatments described herein. For instance, shockwave treatment (i.e. doses of shockwave pulses) may be provided more than once a week, e.g. twice or three times a week. Moreover, the weekly treatment session(s) may be repeated or cycled, e.g. every week, every two weeks, every month or every two months for up to 6 months. Furthermore, these cycles may be repeated, e.g. quarterly, bi-annually, annually etc., e.g. to maintain the beneficial effects, especially in patients with an established infarction where the viability of the remaining cardiomyocytes is at risk. As mentioned above, the efficacy of the treatment may be monitored using cardiac MRI and an appropriate treatment schedule will depend on the subject to be treated and is within the purview of a person of skill in the art.

The DPP-4 inhibitor for use in the combined therapy of the invention may be administered prior to, simultaneously with and/or after administration of the shockwave therapy. In particularly preferred embodiments administration of the DPP-4 inhibitor commences prior to shockwave therapy and is administered throughout the course of the shockwave therapy. Thus, for instance, where shockwave therapy is administered over a course of weeks or months, the DPP-4 inhibitor is administered to the subject continuously over that period, e.g. daily or weekly doses of the DPP-4 inhibitor are administered to the subject over the period of shockwave treatment. Preferably, the DPP-4 inhibitor will be administered prior to the start of shockwave therapy and may be continued after shockwave therapy to maximise the effects. For instance, administration of the DPP-4 inhibitor may commence weeks, e.g. 1-4, 1-3 or 1-2 weeks, prior to the first dose of shockwave pulses and may continue for weeks or months after the last dose of shockwave pulses, e.g. at least 1, 2, 3, 4, 5 or 6 weeks, such as 1, 2, 3, 4, 5 or 6 months after the last dose of shockwave pulses are administered.

The terms "DPP-4 inhibitor" or "DPP-4 antagonist" refer to agents capable of directly or indirectly inhibiting, reducing or blocking the activity or function of DPP-4. For instance, direct inhibitors include agents that interact directly with DPP-4 to inhibit, reduce or block the activity or function of DPP-4. Such agents may work via competitive inhibition, uncompetitive inhibition, on-competitive inhibition or mixed inhibition. Indirect inhibitors do not interact directly with DPP-4. Thus, for instance, indirect inhibitors may inhibit, reduce or block the activity or function of DPP-4 by reducing the expression of the gene encoding the DPP-4 enzyme. In a preferred embodiment, the DPP-4 inhibitor directly inhibits, reduces or blocks the activity or function of DPP-4.

In some preferred embodiments, the term DPP-4 inhibitor refers to a class of oral hypoglycaemic drugs that block DPP-4 activity directly. DPP-4 inhibitors typically are used to block the degradation of glucagon-like peptide 1 (GLP1) and find utility in the treatment of diabetes. Examples of DPP-4 inhibitors include Sitagliptin, Linagliptin, Vildagliptin, Saxagliptin, Gemigliptin, Anagliptin, Teneligliptin, Alogliptin, Trelagliptin, Dutogliptin, Omarigliptinand Lupeol. Thus, in some embodiments, the DPP-4 inhibitor for use in the invention may be Sitagliptin, Linagliptin, Vildagliptin, Gemigliptin, Anagliptin, Teneligliptin, Trelagliptin, Dutogliptin, Omarigliptin, Lupeol or a combination thereof. In a particularly preferred embodiment, the DPP-4 inhibitor for use in the invention may be Sitagliptin, Linagliptin or a combination thereof. In some particularly preferred embodiments, the DPP-4 inhibitor for use in the invention is not Alogliptin or Saxagliptin.

Also included are the salts of such compounds, including both organic and inorganic salts (e.g. with alkali and alkaline earth metals, ammonium, ethanolamine, diethanolamine and meglumine, chloride, hydrogen carbonate, phosphate, sulphate and acetate counterions). Appropriate pharmaceutically and/or physiologically acceptable salts are well described in the pharmaceutical literature. In addition, some of these salts may form solvates with water or organic solvents such as ethanol. Such solvates are also included within the scope of this invention.

The terms "stem cell" and "progenitor cell" as used herein refer to undifferentiated biological cells that can differentiate into specialized cells, i.e. totipotent, pluripotent or multipotent cells. The stem cells are non-embryonic stem cells. In particular, the stem cells or progenitor cells for use in the present invention are capable of differentiating into cardiac stem cells and/or cardiomyocytes. In embodiments where subjects are administered an agent that mobilises stem cells, the stem cells or progenitor cells may be derived or recruited from the peripheral circulation, i.e. blood. Similarly, in embodiments where the stem cells or progenitor cells are harvested or obtained from a subject prior to administration, the stem cells or progenitor cells typically may be harvested or obtained from the peripheral circulation, i.e. blood, of the subject to be treated or a donor subject. However, it will be evident that stem cells or progenitor cells may be harvested or obtained from other appropriate tissues, such as bone marrow. Thus, in some embodiments, the stem cells or progenitor cells are derived from or comprise bone marrow-derived mononuclear cells.

The term "bone marrow-derived mononuclear cells" (BM-MNCs) refers to a population of cells which includes hematopoietic progenitor cells, lymphoid cells (lymphocytes (e.g. T-cells) and plasma cells), monocytes and macrophages. Whilst not wishing to be bound by theory, it is thought that M2 macrophages and hematopoietic progenitor cells may act in combination in the rescue, repair or facilitate the replacement of damaged or dead cardiomyocytes, e.g. resulting from ischaemic reperfusion injury. It is also postulated that T-cells are attracted (recruited) to heart tissue following the shockwave therapy (including combined therapy), which would create an environment conducive for cell survival.

Thus, in preferred embodiments, it may be useful to use a population or combination of stem cells, i.e. the stem cells or progenitor cells comprise a population of cell comprising hematopoietic progenitor cells and optionally one or more cell types selected from lymphoid cells (lymphocytes, e.g. T-cells), monocytes and macrophages. In some instances, it may be useful to use a subset of BM-MNCs. Thus, in some embodiments the stem cells or progenitor cells are or comprise hematopoietic progenitor cells and optionally macrophages, particularly M2 macrophages.

The term "M2 macrophages" (also referred to as alternatively activated macrophages) broadly refers to macrophages that function in constructive processes like wound healing and tissue repair. M2 macrophages may also turn off damaging immune system activation by producing anti-inflammatory cytokines like IL-10. M2 macrophages produce high levels of IL-10, TGF-beta and low levels of IL-12. Notably, M2 macrophages do not constitute a uniform population and often are further subdivided into M2a, M2b and M2c categories. M2a macrophages are involved in the Th2 type immune response, e.g. against parasites. M2b macrophages are considered immunity-regulating and are induced by IL-1, LPS and immune complexes. M2c macrophages are induced in the presence of IL-10 and TGF-b. They are often referred to as deactivated or anti-inflammatory and are known to be involved in tissue repair and remodelling. They produce large amounts of IL-10 and TGF-beta and express multiple receptors, such as: CD163, CD206, RAGE and other scavenger receptors. Thus, in some embodiments, the M2 macrophages are M2c macrophages.

The term "pharmacological stem cell mobiliser" refers to agents capable of directly or indirectly inducing, enhancing or increasing the number of stem cells in the peripheral circulation of a subject. In particular, the pharmacological stem cell mobiliser may be a hematopoietic stem cell mobiliser. Various agents are known in the art, such as parathyroid hormone, G-CSF, ancestim, Mozobil, plerixafor and Stemgen and any such agent may be used in the uses of the invention. In a preferred embodiment the pharmacological stem cell mobiliser is parathyroid hormone or a fragment thereof.

Parathyroid hormone (PTH), also called parathormone or parathyrin, is a hormone secreted by the parathyroid glands. PTH is secreted by the chief cells of the parathyroid glands as a polypeptide containing 84 amino acids, which is a prohormone. Notably, the effective hormone-receptor interaction requires solely the 34-N-terminal amino acids of the prohormone. Thus, the fragment of PTH for use in the invention comprises at least the 34-N-terminal amino acids of PTH. In some embodiments, the fragment comprises or consists of teriparatide. Teriparatide is a recombinant form of PTH consisting of the 34-N-terminal amino acids of PTH.

The term "engraftment" refers to the incorporation of grafted tissue into the body of the host. Thus, in the context of the present invention, engraftment refers to the incorporation of stem cells into the heart tissue of the subject to be treated. The stem cells may be endogenous to the subject to be treated, e.g. mobilised as a result of the administration of a stem cell mobiliser, such as parathyroid hormone. Alternatively, the stem cells may be exogenous, i.e. administered separately, e.g. obtained or harvested from the subject to be treated or a donor subject, optionally modified *in vitro,* and subsequently administered to the subject to be treated.

The term "heart failure" as used herein includes any condition characterised by impaired cardiac function, specifically impaired ventricular function (ventricular dysfunction), either due to reduced pump action (systolic dysfunction) or reduced filling (diastolic dysfunction). Systolic dysfunction may be described as a condition of ventricular contractile dysfunction. Inadequate ventricular emptying is seen. Diastolic dysfunction may be described as resistance to ventricular filing. Heart failure may thus be seen as a ventricular condition or condition of ventricular failure.

The heart failure may be left-sided (left ventricular involvement or dysfunction) or right-sided (right ventricular involvement or dysfunction) or it may involve both the sides of the heart (both right and left ventricles). Heart failure implies impaired function of the myocardium of the heart. Thus, in some embodiments, the therapeutic uses disclosed herein may improve the function of the myocardium of the heart. Notably however, in some patients, especially in the early phase of heart failure, the ejection fraction (discussed below) could be within normal parameters, e.g. 50% or higher, i.e. 50-70%. For instance, various compensatory mechanisms may increase the stroke volume such that the ejection fraction is not immediately reduced (e.g. by alteration of the preload, afterload, systemic vascular resistance or other compensatory mechanism). Thus, in some embodiments, the therapeutic uses disclosed herein may maintain the function of the myocardium of the heart.

Particularly, chronic forms of heart failure (i.e. chronic heart failure) are concerned.

Thus, heart failure can be defined as a disorder which may result from any condition that reduces the ability of the heart to pump blood. Often the cause is decreased contractility of the myocardium resulting from diminished coronary blood flow (e.g. heart failure caused by coronary artery disease (CAD) or coronary ischemic disease), but failure to pump adequate quantities of blood can also be caused by damage to heart valves, external pressure around the heart, primary cardiac muscle diseases (e.g. idiopathic dilated cardiomyopathy) or any other abnormality which makes the heart a hypoeffective pump. As mentioned above, chronic heart failure is particularly concerned.

Thus, included is heart failure caused by or resulting from ischaemic heart disease (ischaemic cardiomyopathy), particularly chronic ischaemic heart disease (e.g. chronic myocardial ischemia). Thus, heart failure to be treated by the uses of the invention is chronic or ischaemic heart failure. In particularly preferred embodiments, heart failure to be treated by the uses of the present invention is a result of cardiac ischaemic reperfusion injury, e.g. caused by myocardial infarction.

As described above, cardiac ischaemic reperfusion injury refers to the damage to cardiac tissue caused when blood supply returns to the cardiac tissue after a period of ischemia or lack of oxygen (anoxia, hypoxia). The absence of oxygen and nutrients from blood during the ischemic period creates a condition in which the restoration of circulation and rapid elevation of oxygen levels (i.e. normoxia or hyperoxia) results in inflammation and oxidative damage through the induction of oxidative stress rather than restoration of normal function. This in turn may result in apoptosis of cardiomyocytes and a subsequent impairment of the myocardium of the heart.

"Hypoxia" refers to a condition of low oxygen tension, typically in the range 1-5% O₂, and is often found in the central region of tumours due to poor vascularisation. "Anoxia" refers to a substantial absence of oxygen, e.g. oxygen tensions of less than 0.5% O₂, such as less than 0.1% O₂. "Normoxia" refers oxygen tensions between 10-21 %. "Hyperoxia" refers of oxygen tensions above 21%.

Heart failure may be manifest in two ways: (1) by a decrease in cardiac output or (2) by a damming of blood in the veins behind the left or right heart. The heart can fail as a whole unit or either the left side or the right side can fail independently of the other. Either way, heart failure may lead to circulatory congestion and this has in the past been referred to as congestive heart failure.

Heart failure can be divided into two phases, acute heart failure (short term and unstable) and chronic heart failure (long term and relatively stable). The division between the two is difficult to define precisely, but generally acute heart failure is the stage of failure which occurs immediately after heart damage (i.e. has a rapid onset and short course) and is associated with instability in cardiac function and circulation, for example a sudden drop in cardiac output. Providing the acute phase is not so severe as to result in death, the sympathetic reflexes of the body are immediately activated and can compensate for the sudden loss in cardiac function. Such compensation can often be so effective and rapid that it is possible that no noticeable effect on the subject will be felt if a subject remains calm.

After the first few minutes of an acute heart attack, a prolonged secondary state begins. This is characterised by a retention of fluid by the kidneys and by the progressive recovery of the heart over a period of several weeks to months up until the point at which the cardiac condition stabilises. This phase of stability is known as chronic heart failure. Although the heart has compensated and stabilised it is still weak and may become progressively weaker.

Thus, while the acute phase of heart failure is over relatively quickly, the stability associated with the chronic phase of heart failure can take a matter of months to develop. Generally, a patient exhibiting symptoms of heart failure for greater than 3 months or more preferably greater than 6 months can be regarded as having chronic heart failure, providing that no further symptoms of acute heart failure have occurred during this 3 month or 6 month period.

This means therefore that although symptoms vary largely between subjects, subjects with heart failure, and particularly chronic heart failure, characteristically have a reduced cardiac function, and in particular reduced ventricular function. The most common manifestation of reduced cardiac performance is systolic dysfunction. Thus, the heart failure may be systolic heart failure. For example, such subjects display a reduced ventricular ejection fraction, particularly a reduced left ventricular ejection fraction (LVEF) when compared to a "normal" subject who has not suffered from heart failure. In normal persons left ventricular ejection fraction is usually above 60% (typically between 55%-70%), while an ejection fraction less than 45%, particularly less than 40%, is characterized as systolic dysfunction. Thus, a LVEF of less than 45%, particularly less than 40%, is characteristic of reduced heart function in subjects with heart failure, particularly chronic heart failure. Typically, an ejection fraction of 35%-45% may be characterised as mildly impaired heart function. An ejection fraction of 25%-35% may be characterised as moderately impaired heart function. An ejection fraction of less than 25% may be characterised as severely impaired heart function. Subjects with an ejection fraction of less than 15% may be characterised as having end-stage heart failure and may be candidates for a heart transplant. Subjects with an ejection fraction of less than 5% are not expected to survive long-term.

Less common than systolic dysfunction is diastolic dysfunction in which the ejection fraction is relatively preserved (e.g. left ventricular EF>40%) or normal, but where ventricular filling, e.g. left ventricular filling, is reduced.

Preferred subjects for treatment according to the present invention include those with a LVEF of less than 40% (LVEF <0.40). In some embodiments, subjects may be defined as those with an LVEF of less than 35% (LVEF <0.35). However, as mentioned above, some subjects in the early phase of heart failure may have a normal LVEF, e.g. 45% or higher. Thus, in some embodiments, subjects for treatment according to the present invention include those with a LVEF of less than 60% (LVEF <0.60), preferably less than 55% (LVEF <0.55), such as less than 50% (LVEF <0.50) or less than 45% (LVEF <0.45).

Other characteristics of reduced cardiac function which may be seen in heart failure include a reduced right ventricular ejection fraction, reduced exercise capacity and impaired haemodynamic variables such as a decreased cardiac output, increased pulmonary arterial pressure and increased heart rate and low blood pressure.

The New York Heart Association (NYHA) classification system divides heart disease into four classes, depending on the severity of disease. NYHA class I: Patients with cardiac disease but without resulting limitations of physical activity, e.g. no shortness of breath, fatigue or heart palpitations with ordinary physical activity; Class II: Patients with cardiac disease resulting in slight limitation of physical activity, e.g. shortness of breath, fatigue or heart palpitations with ordinary physical activity, but patients are comfortable at rest. Class III: Patients with cardiac disease resulting in marked limitation of physical performance, e.g. shortness of breath, fatigue, or heart palpitations with less than ordinary physical activity, but patients are comfortable at rest. Class IV: Patients with cardiac disease resulting in inability to carry on any physical activity without discomfort, e.g. shortness of breath, fatigue or heart palpitations with any physical exertion and symptoms may be present even at rest.

The invention may be used for the treatment or prophylaxis of all classes of heart failure but particularly the classes II-IV or subjects in classes III-IV. Thus, the patient or subject may be in any one or more of classes I to IV, but preferably they are in classes II-IV or III-IV.

Thus, the present invention may be used for the treatment or prevention (prophylaxis) of any kind of heart failure, irrespective of cause or aetiology. The resistance of the heart to heart failure may be increased. The present invention may thus be used to treat established or symptomatic or overt heart failure, particularly chronic heart failure, but including also acute heart failure or heart failure which is evolving or developing, including incipient heart failure or heart failure which is asymptomatic. It may also be used to prevent or delay the onset of heart failure or to prevent, limit or reduce the development of heart failure, for example to reduce or limit the extent or degree to which the heart failure develops or to reduce the susceptibility of the heart to heart failure. Thus, for example the development of terminal heart failure may be delayed. Thus, alternatively viewed, the invention may be used to improve the prognosis of a subject with, or at risk of, heart failure.

As mentioned above, the heart failure, particularly chronic heart failure, to be treated according to the present invention may result from any cause, e.g. may be the result of a primary disease or may be secondary to another disease. In one embodiment of the invention the heart failure to be treated is chronic heart failure secondary to either idiopathic dilated cardiomyopathy (IDCM) and/or coronary ischemic disease (coronary artery disease - CAD).

Particularly, in a further preferred embodiment, the heart failure to be treated according to the invention is post-infarction heart failure or ischaemic heart failure.

Other types of heart failure which may be treated according to the invention include heart failure induced by a constantly increased after load, such as hypertensive heart failure. Heart failure arising from any other cause, for example as mentioned above, is included within the scope of this invention.

As noted above, subjects to be treated according to the present invention may exhibit symptomatic heart failure. Symptoms of heart failure are listed above and may include weight gain, swelling of feet and ankles or abdomen, pronounced neck veins, loss of appetite, indigestion, nausea and vomiting, shortness of breath with activity or after lying down, difficulty sleeping, fatigue, weakness or faintness, palpitations, irregular or rapid pulse, decreased alertness or concentration, cough, decreased urine output, and need to urinate at night, although not all or any of these symptoms may be present with heart failure.

Other characteristics of heart failure which may be manifest include reduced ejection fractions (LV and/or RV), reduced exercise capacity and impaired haemodynamic variables such as those listed above.

The subjects may be receiving standard treatment(s) for heart failure, including standard pharmacological treatments such as one or more of beta blockers, ACE inhibitors, ARBs, aldosterone antagonists, diuretics, antihypertensives, cardiac glycosides, angiotensin receptor-neprilysin inhibitor (ARNI) combination therapy (e.g. Valsartan/sacubitril) etc. Particularly the subjects may or may not be receiving beta blockers. For instance, the subject may be receiving treatment with an NPR-A agonist, such as BNP, e.g. recombinant BNP or an analogue or derivative thereof, or another natriuretic peptide or an analogue or derivative thereof.

The development or progression of heart failure may be associated with ventricular remodelling, particularly left ventricular remodelling, which manifests as gradual increases in left ventricular end-diastolic and end-systolic volumes, wall thinning, and a change in chamber geometry to a more spherical, less elongated shape. This process is usually associated with a continuous decline in ejection fraction. In one embodiment of the invention remodelling, and particularly ventricular, preferably left ventricular, remodelling may be reduced or prevented.

Remodelling may lead to ventricular dysfunction, particularly left ventricular dysfunction. In another embodiment of the invention, ventricular dysfunction is treated or prevented.

As used herein "treatment" refers to reducing, alleviating, ameliorating or eliminating the disease (which term includes any disease, condition or disorder), or one or more symptoms thereof, which is being treated (e.g. the heart failure or a symptom thereof), relative to the disease or symptom prior to the treatment. Treatment may include an improvement or increase in cardiac function or performance, and in particular ventricular function or performance, more particularly left ventricular function or performance.

"Prophylaxis" or "prevention" as used herein refers to delaying, limiting or reducing the disease or the onset of the disease, or one or more symptoms thereof, for example relative to the disease or symptom prior to the prophylactic or preventative treatment. Prophylaxis thus explicitly includes both absolute prevention of occurrence or development of the disease or symptom, or reduction or limitation of the development or progression of the disease or symptom.

The subject or patient of treatment or prophylaxis may be any human or non-human animal subject, but preferably will be a mammal, for example a human or any livestock or domestic animal, e.g. mice, rats, pigs, cats, dogs, sheep, rabbits, horses, cows or monkeys. Most preferably the subject will be a human subject.

Given the nature of most forms of heart disease it is not to be expected that "treatment" in accordance with the present invention will result in a complete cure of the heart failure in question. Rather, "treatment" in accordance with the present invention includes an improvement or alleviation of any of the symptoms associated with the heart failure and also an improved quality of life for a patient and, ultimately a prolonged lifetime and improved survival, i.e. an improved prognosis.

"Treatment" in accordance with the present invention also includes an improvement or increase of the functionality of the heart or, in other words a long-term improvement or increase in cardiac function or performance. In particular, treatment in accordance with the present invention may result in an improvement or increase in any one or more of the symptoms and functional parameters associated with heart failure and in particular the symptoms and parameters relating to ventricular and particularly left ventricular function.

An important symptom and parameter associated with improved cardiac function in heart failure is an increase in ventricular ejection fraction and in particular left ventricular ejection fraction (LVEF). This can be assessed by standard methods well known and documented in the art, for example by echocardiography ("echo", i.e. a cardiac echo, which is a sonogram of the heart), electrocardiogram (ECG), ECG synchronized gated radionuclide ventriculography (MUGA scan), angiography or magnetic resonance imaging (MRI), and is normally carried out when the subject is at rest. An improvement in LVEF has been found to be associated with improved survival amongst heart failure patients. Thus, this is an important and advantageous parameter to be improved in subjects treated in accordance with the present invention. RVEF may also be increased.

Whilst an improvement of LVEF is particularly important for the overall improvement of heart function, a number of other parameters associated with cardiac performance may be improved in accordance with the present invention. One of these is a significant improvement in overall clinical status and thus clinical performance as evaluated by NYHA functional class. In other words, the NYHA functional class of a patient may be reduced after treatment in accordance with the present invention. Such a clinical evaluation may normally be carried out by a trained cardiologist.

Other parameters include exercise capacity for example, as measured by peak oxygen uptake and peak work load. As indicated above, a decreased exercise capacity is an inconvenient and potentially debilitating symptom of many heart failure patients. Methods for measuring exercise capacity are well known and documented in the art. For example, exercise testing can be carried out using an electrically braked bicycle ergometer. An exemplary protocol might consist of a starting work rate of 20 W increasing by 20 W every second minute until exhaustion (defined as an inability to keep the pedalling rate steady at 60 rpm). Oxygen uptake (VO2) can be measured using for example the EOS/SPRINT system. Peak VO2, is taken as the highest VO2 observed. Another example of exercise testing is the 6-minute walk test, where the patient is asked to walk as long as possible during 6-minutes under standardised conditions, and the walked distance represents a measure of exercise capacity.

Hemodynamic echocardiographic parameters and variables may also be assessed to indicate improved cardiac function. For example, improved cardiac function may be indicated by a decrease in pulmonary capillary wedged pressure and/or in pulmonary artery pressure, and/or an increase in peak heart rate, peak systolic blood pressure and mitral velocity deceleration time. Echocardiographic variables may conveniently be measured by echocardiography carried out by a trained cardiologist and haemodynamic variables can conveniently be assessed by right-sided heart catheterization according to standard techniques.

Another important variable which may be assessed is the plasma level of Nt-proANP or Nt-proBNP. Increased or generally high levels of Nt-proANP or Nt-proBNP have been recognised as markers of cardiac dysfunction. Moreover, levels of Nt-proANP have been shown in the past to correlate with pulmonary artery pressures in heart failure and provide important prognostic information in heart failure patients. Levels of Nt-proANP or Nt-proBNP in a blood sample can be measured in a number of ways well known and documented in the art, for example by radioimmunoassay. Prior to the immunoassay, plasma is separated from a blood sample taken from the patient again by methods well known and documented in the art.

The above described "improvement" or "increase" in the symptoms, parameters and/or prognosis of the subject includes any measurable improvement or increase when the parameter in question is compared with the equivalent parameter in a non-treated individual or when the parameter in question is compared with the equivalent parameter in the same individual taken at an earlier time point (e.g. comparison with a "base line" level). Preferably the improvement or increase will be statistically significant. Especially preferably the improvement or increase in the symptoms, parameters and/or prognosis will be associated with the improved health of the patient concerned and more preferably a prolonged survival.

Methods of determining the statistical significance of differences in parameters are well known and documented in the art. For example, herein a parameter is generally regarded as significant if a statistical comparison using a two-tailed significance test such as a Student t-test or Mann-Whitney U Rank-Sum test shows a probability value of <0.05.

Thus, the present invention may be used in the improvement of cardiac function, particularly cardiac function in heart failure. More specifically, in the long-term, the present invention may be used to increase ventricular function, particularly left ventricular function (e.g. LVEF), more particularly in heart failure.

The present invention may result in the reduction of plasma Nt-proANP or Nt-proBNP levels. As mentioned above, a reduction in the plasma levels of Nt-proANP or Nt-proBNP is an indicator of improved cardiac function and performance. "Reduction" as used herein includes any measurable reduction when the parameter in question is compared with the equivalent parameter in a non-treated individual or when the parameter in question is compared with the equivalent parameter in the same individual taken at an earlier time point (e.g. comparison with a "base line" level). Preferably the reduction is statistically significant as discussed above. Especially preferably the reduction in the levels of Nt-proANP or Nt-proBNP will be associated with an improved feeling of health in the patient concerned and more preferably a prolonged survival.

In one aspect the patient or subject may be identified as in need of treatment or prophylaxis of heart failure (e.g. as suffering from heart failure, or as being at risk of developing, or susceptible to, heart failure), before the combined therapy of the invention is administered.

Such identification can be on the basis of symptoms and/or parameters which are indicative of heart failure, or risk of heart failure, as discussed above.

As discussed above, cardiac performance may be increased following the administration of the combined therapy of the invention. Accordingly, the various aspects of the present invention as presented and discussed above may further include assessing the subject being treated for an improvement in cardiac performance, or in the heart failure or symptom thereof following administration of the combined therapy of the invention. As discussed above, this assessment may be an assessment for an improvement in cardiac performance or function, particularly ventricular and especially left ventricular, performance or function or for an improvement in any symptom or parameter of heart failure, as discussed above.

Where the subject is at risk of developing a heart failure, or is susceptible to heart failure, the subject may be assessed for one or more factors which are risk factors for heart failure. For example, these may be ischaemic disease or conditions, e.g. coronary artery disease, cardiomyopathy, hypertension, valvular disease, congenital heart defects or any other predisposing condition or factor known in the art or described or mentioned above.

Following administration of the combined therapy of the invention, the subject may be assessed for the development of heart failure or for one or more risk factors for heart failure.

The shockwave therapy is used in combination with a pharmacological agent, i.e. a DPP-4 inhibitor and optionally a mobiliser of stem cells (e.g. parathyroid hormone), wherein the pharmacological agent may be conveniently formulated in a pharmaceutical composition for use according to the present invention. A pharmaceutical composition refers to a composition comprising a pharmacological agent (i.e. a pharmacologically active agent or ingredient), e.g. a DPP-4 inhibitor and/or mobiliser of stem cells (e.g. parathyroid hormone) with at least one pharmaceutically acceptable carrier, diluent or excipient. Thus, for instance, the present invention may be viewed as providing a pharmaceutical composition for use in the treatment or prevention (prophylaxis) of heart failure, or in the treatment of cardiac ischaemic reperfusion injury, in a subject, wherein said subject has been administered extracorporeal cardiac shockwave therapy and wherein said composition comprises a DPP-4 inhibitor together with at least one pharmaceutically acceptable carrier, and is for administration prior to, simultaneously with, and/or after administration of said shockwave therapy.

The appropriate content of active ingredient (a pharmacological agent, i.e. a pharmacologically active agent or ingredient) in such compositions may be determined according to principles and procedures routine in the art and may readily be determined by the skilled practitioner. Thus, for example, the active ingredient in such compositions may comprise from 0.05% to 99% by weight of the formulation, for example from 0.1% to 1.0% or around 0.5%. The concentration of active ingredient in the formulation will depend on the type of formulation. For example, enteral products (e.g. tablets and capsules) can typically have 5% to 50% active ingredients by weight, whereas parenteral formulations usually have a lower concentration of active compound e.g. 0.1% to 3% active ingredient by weight, for example in an injection solution.

By "pharmaceutically acceptable" is meant that the ingredients must be compatible with other ingredients of the composition as well as physiologically acceptable to the recipient.

A pharmaceutical composition may be formulated according to any of the conventional methods known in the art and widely described in the literature. Thus, the active ingredient (e.g. a DPP-4 inhibitor) may be incorporated, optionally together with other active substances, with one or more conventional carriers, diluents and/or excipients, to produce conventional galenic preparations which are suitable or can be made suitable for oral, subcutaneous, intramuscular, intravenous or any other administration such as powders, sachets, cachets, elixirs, suspensions, emulsions, solutions, syrups, ointments, sterile injectable solutions, sterile packaged powders, and the like. A pharmaceutical composition comprising an active ingredient (e.g. parathyroid hormone) may be prepared in a form appropriate for infusion or injection into a patient. Such infusion or injection is preferably intramuscular (i.m.) but may also be given subcutaneously (s.c.) or intravenously (i.v.).

Preferably, the compositions comprising the DPP-4 inhibitor may be provided in a form adapted for oral administration. For instance, pharmaceutical forms may include plain or coated tablets, capsules, suspensions and solutions containing the active component (e.g. DPP-4 inhibitor) optionally together with one or more inert conventional carriers and/or diluents.

Preferably, the compositions comprising the pharmacological agent for mobilising stem cells (e.g. parathyroid hormone) may be provided in a form adapted for parenteral administration, particularly intramuscular or intra-abdominal administration. For instance, pharmaceutical forms may include sterile injectable solutions or suspensions optionally together with one or more inert conventional carriers and/or diluents.

Examples of suitable carriers, excipients, and diluents are lactose, dextrose, sucrose, maltose, glucose, sorbitol, mannitol, starches, gum acacia, calcium phosphate, aglinates, tragacanth, gelatin, calcium silicate, microcrystalline cellulose, polyvinylpyrrolidone, cellulose, water syrup, water, water/ethanol, water/ glycol, water/polyethylene, glycol, propylene glycol, methyl cellulose, methylhydroxybenzoates, propyl hydroxybenzoates, talc, magnesium stearate, mineral oil or fatty substances such as hard fat or suitable mixtures thereof. The compositions may additionally include lubricating agents, wetting agents, emulsifying agents, suspending agents, preserving agents, sweetening agents, flavouring agents, and the like. The compositions may be formulated so as to provide quick, sustained or delayed release of the active ingredient after administration to the patient by employing procedures well known in the art.

Solubilizing and/or stabilizing agents may also be used, e.g. cyclodextrins (CD) α, β, γ and HP-β cyclodextrin.

Suitable doses will vary from patient to patient and can be determined by the physician in accordance with the weight, age and sex of the patient, mode of administration, and the severity of the condition and also the particular active ingredient used for treatment. Exemplary unit doses for oral administration (e.g. the DPP-4 inhibitor) may contain 1 to 250 mg, of the active ingredient, although it will be understood that this may of course vary, depending on the particular antagonist used etc. For instance, a unit dose of sitagliptin may be in the range of 100mg, e.g. 75-125mg, whereas a unit dose of linagliptin may be in the range of 5mg, e.g. 1-10mg. The daily dose for oral administration may for example be in the range of approximately 0.01 to 10 mg/kg/day, e.g. 0.1 to 5 mg/kg/day, for example 0.1 to 2 mg/kg/day. For example, a 70 kg adult would receive a daily dose of 1 to 700 mg or 0.7 to 700 mg, more usually 1 to 350 mg or 7 to 350 mg for example 7 to 140 mg. For parenteral (e.g. intravenous or intramuscular) administration (e.g. parathyroid hormone) exemplary unit doses may be between 0.1 µg and 100 µg, for example between 0.1 µg and 50µg, such as 1-40, 2-35, 3-40, 4-35 or 5-25µg, e.g. 20µg. The daily dose for parenteral administration may for example be in the range of approximately 0.001 to 1 µg/kg/day, e.g. 0.001 to 0.5 µg/kg/day, for example 0.001 to 0.3 µg/kg/day. For example, a 70 kg adult would receive a daily dose of 0.01 to 70 µg or 0.07 to 70µg, more usually 0.07 to 35 µg or 0.7 to 35 µg for example 0.7 to 21 µg.

The improvements seen in patients treated in accordance with the present invention may be seen after a few days, weeks or months depending on the individual patient. Once the initial improvement is seen, continued improvement over the subsequent weeks and months may also occur. As indicated above, treatment can be continued for as long as is desired or is necessary.

The combination therapy of the present invention may be in place of, or in addition to (i.e. in combination with) the use of other drugs for treatment of heart failure. Thus, other drugs known to treat heart failure might be included in the pharmaceutical compositions described above or may be administered separately, in a manner appropriate for the drug concerned.

Suitable additional or supplementary drugs or agents for treatment of heart failure are well known and documented in the art and include known drugs for use in the treatment of heart disorders. For example, diuretics, vasodilators, inotropic drugs such as digoxin or digitoxin, or other compounds such as anticoagulants, β blockers, angiotensin II blockers, angiotensin converting enzyme inhibitors, angiotensin receptor-neprilysin inhibitor (ARNI) combination therapies (e.g. Valsartan/sacubitril) or aldosterone antagonists may be used.

In some embodiments, it may be useful to administer sugar, e.g. glucose, to the subjects to be treated. In particular, the combination therapies of the invention utilise DPP-4 inhibitors, which block the degradation of glucagon-like peptide 1 (GLP-1). GLP-1 increases insulin secretion while inhibiting glucagon release, thereby lowering plasma glucose levels. Accordingly, in some embodiments the supplementary or additional agent for administration to the subject to be treated is glucose.

The additional or supplementary drugs or agents may be separately formulated, for administration alongside, e.g. at the same time or sequentially with, or at separate intervals from, the active agents for use in the combined shockwave therapy of the invention (e.g. DPP-4 inhibitor and/or pharmacological mobiliser of stem cells, e.g. parathyroid hormone).

The additional or supplementary drugs or agents may thus be provided with the active agents for use in the combined shockwave therapy of the invention in the form of a kit. Such a kit may comprise, for example separate containers for (e.g. comprising or containing) the active agents for use in the combined shockwave therapy of the invention and additional or supplementary drugs or agents, respectively, optionally together with instructions for use.

For instance, the present invention may be seen to provide a kit or product containing:
(i) a DPP-4 inhibitor or pharmaceutical composition comprising said inhibitor and at least one pharmaceutically acceptable carrier, diluent or excipient; and
(ii) a pharmacological agent for mobilising stem cells, such as parathyroid hormone or fragment thereof, or a pharmaceutical composition comprising said pharmacological agent and at least one pharmaceutically acceptable carrier, diluent or excipient; and optionally
(iii) an additional agent, e.g. an agent for treatment of heart failure or glucose, or a pharmaceutical composition comprising said additional agent and at least one pharmaceutically acceptable carrier, diluent or excipient,
wherein (i), (ii) and optionally (iii) of the kit or product described above are provided as a combined preparation for simultaneous, sequential or separate use in:
(a) treating or preventing heart failure; or
(b) treating cardiac ischaemic reperfusion injury,
in a subject, wherein said subject has been administered extracorporeal cardiac shockwave therapy and wherein (i) and/or (ii) are for administration prior to, simultaneously with, and/or after administration of said shockwave therapy.

The invention will be further described with reference to the following nonlimiting Examples with reference to the following drawings in which:
Figure 1 shows a bar chart showing the percentage of viable rat cardiomyocytes under various treatment conditions (error bars represent the standard deviation, **** p≤0.001, "ns" no significance).
Figure 2 shows bar charts of the relative amount (fold change) of (A) SDF-1 gene expression and (B) MCP-1 gene expression in human ventricular tissue four hours after various shockwave treatments (* p >0.05, ** p≤0.05, *** p≤0.001 and **** p ≤0.0001).
Figure 3 shows bar charts of the relative amount (fold change) of (A) ANGP-1 (Angiopoietin) gene expression and (B) VEGFA (Vascular endothelial growth factor A) gene expression in human ventricular tissue four hours after various shockwave treatments (* p >0.05, ** p≤0.05, *** p≤0.001 and **** p ≤0.0001).
Figure 4 shows bar charts of the relative amount (fold change) of (A) NOS-3 (Nitric Oxide Synthase 3) gene expression and (B) TAC-1 (Tachykinin Precursor 1) gene expression in human ventricular tissue four hours after various shockwave treatments (* p >0.05, ** p≤0.05, *** p≤0.001 and **** p ≤0.0001).
Figure 5 shows graphs of the relative amount (fold change) of SDF-1 gene expression over time in (A) Human umbilical vein endothelial cells (HUVECs) and (B) human cardiac fibroblasts after various shockwave treatments (* p >0.05, ** p≤0.05, *** p≤0.001 and **** p ≤0.0001).
Figure 6 shows graphs of the relative amount (fold change) of (A) VEGFA gene expression and (B) MCP-1 (Monocyte chemotactic protein 1) gene expression over time in human cardiac fibroblasts after various shockwave treatments (* p >0.05, ** p≤0.05, *** p≤0.001 and **** p ≤0.0001).
Figure 7 shows bar charts of the relative amount (fold change) in AKT phosphorylation in rat cardiomyocytes under various conditions, wherein (A) shows the relative change of normalised p-AKT308 and (B) show the relative change of the ratio of p-AKT308 and unphosphorylated AKT.
Figure 8 shows bar charts of the relative amount (fold change) of (A) SDF-1 gene expression and (B) VEGFA gene expression in rat cardiomyocytes four hours after various shockwave treatments.
Figure 9 shows photomicrographs of frozen tissue sections from rat hearts subjected to the following treatments: (A) no treatment; (B) extracorporeal cardiac shockwave treatment and daily water gavages; (C) extracorporeal cardiac shockwave treatment and daily DPP4i gavages; and (D) daily DPP4i gavages, as described in Example 7. The circles show areas of brown deposits that are evidence of the presence of SDF-1.
Figure 10 shows photographs of fixed rat hearts subjected to the following treatments: (A) no treatment; (B) extracorporeal cardiac shockwave treatment and daily water gavages; (C) extracorporeal cardiac shockwave treatment and daily DPP4i gavages; and (D) daily DPP4i gavages, as described in Example 7.

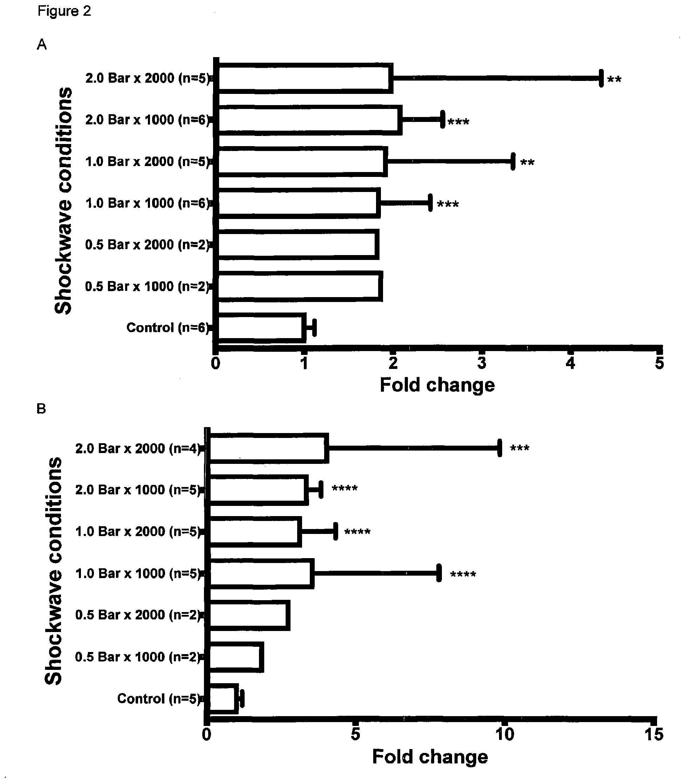

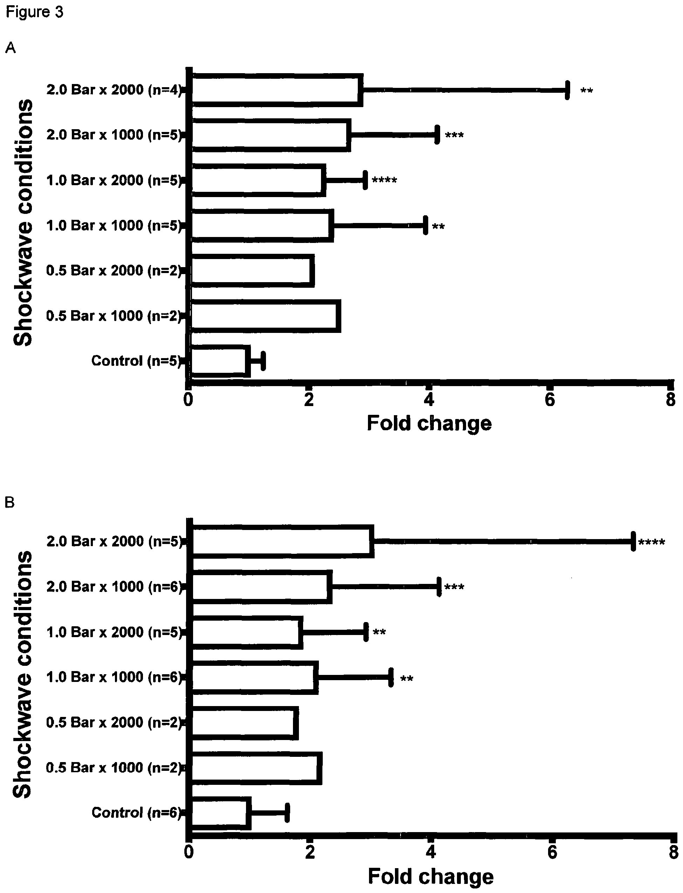

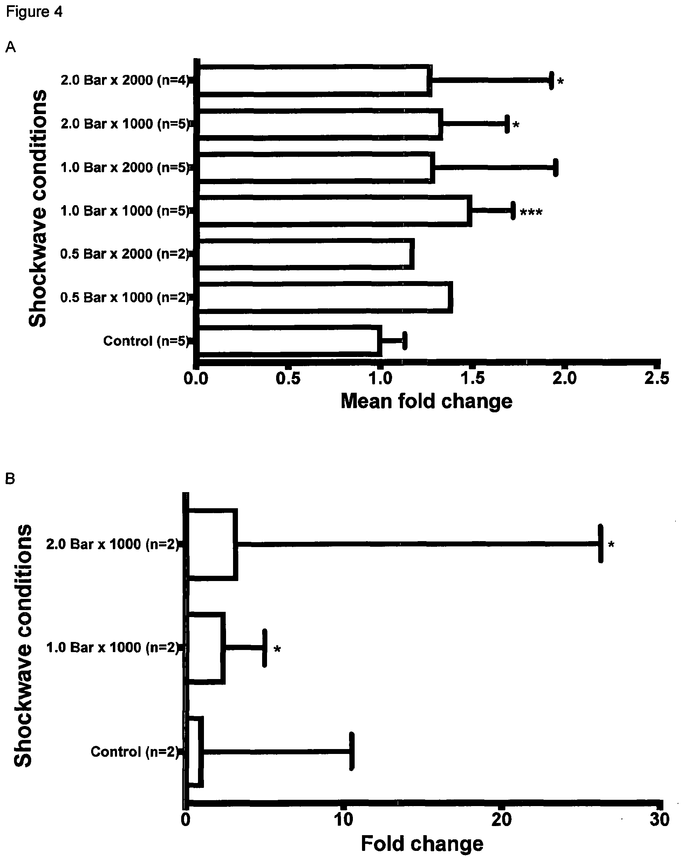

The error bars in Figures 2-6 and 8 represent the 95% confidence interval (CI).

### Examples

### Example 1: Effect of shockwave treatment on hypoxia induced apoptosis in rat cardiomyocytes

Primary rat cardiomyocytes were isolated using the Langendorff method, where the explanted hearts were retrogradely perfused through the aorta with oxygenated low calcium solutions and collagenase solutions. The hearts were then diced and agitated in collagenase solutions to release the cells. The cells were washed and the cardiomyocytes were then positively selected using low-speed centrifugation and by adherence to laminin-coated culture plates (Petri dishes).

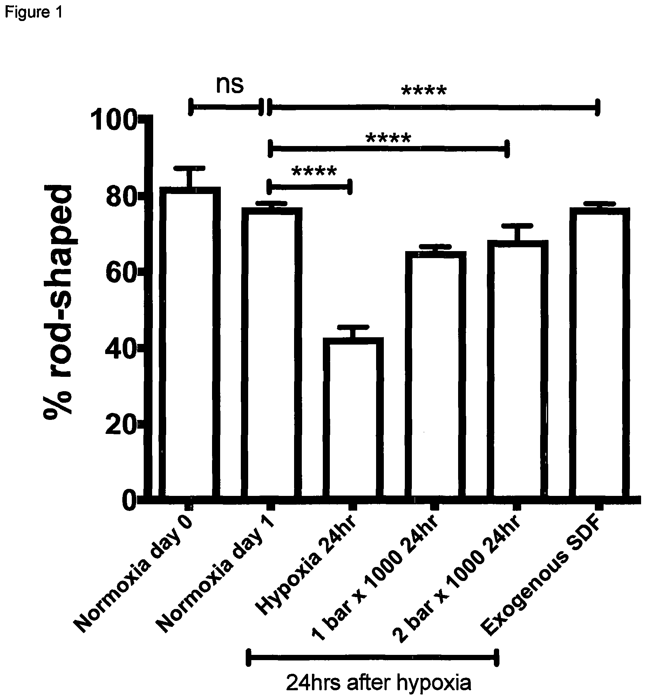

The rat cardiomyocytes were exposed to severe hypoxia for 30 minutes (0% O₂, 100% N₂). The groups receiving shockwave treatments (1000 pulses of 1 bar or 2 bar) were treated immediately upon return to normoxia. Shockwaves were delivered from the underneath the Petri dishes via direct contact between the shockwave applicator, on the handpiece of Swiss Dolarclast (EMS) shockwave system, coupled with ultrasound gel. Notably the group receiving exogenous SDF received no shockwave treatment upon returned to normoxia. The viability of the cells was assessed by counting the number of rod-shaped and round-shaped cells (wherein rod-shaped cells are viable) in triplicate 24 hours after shockwave or SDF treatment. The results are shown in Figure 1 and demonstrate that post-conditioning using shockwave therapy immediately after hypoxia increases cardiomyocyte viability relative to untreated controls, which suggests that shockwave therapy may attenuate ischemic reperfusion injury.

### Example 2: Effect of shockwave treatment on gene expression in human ventricular tissue

Explanted human cardiac ventricular tissue was cut into small pieces and individually cultured in 24-well plates in M199 media. Exposure to various shockwave conditions was performed by temporarily placing the tissue pieces in 1.5ml Eppendorf tubes in M199 media. The shockwave applicator described in Example 1 was directly coupled to the tubes using ultrasound gel. The tissue pieces were then returned to their respective culture media in the incubator (37°C and 5% CO₂) for the 4-hour time points and then stored in RNA-Later at -80°C. In batches, the samples were completely homogenised in Trizol using a power homogenizer. The RNA was purified using chloroform and commercial spin columns. The quality of RNA was inspected using a Nanodrop spectrophotometer and reverse transcription reactions were performed. The gene expression of SDF1 (Stromal derived factor 1), VEGFA (Vascular endothelial growth factor A), MCP1 (Monocyte chemotactic protein 1), ANGP1 (Angiopoietin), TAC1 (Tachykinin Precursor 1) and NOS3 (Nitric Oxide Synthase 3) was assessed using Taqman probes in TaqMan Gene Expression Master Mix normalised using GAPDH, on the Applied Biosystems^{™} 7900HT Fast Real-Time PCR System. The fold changes were calculated using the ΔΔCT method. The results are shown in Figures 2-4 and demonstrate that there is a statistically significance difference between the untreated controls and the 4-hour time points for all of the genes measured.

### Example 3: Effect of shockwave treatment on SDF-1 gene expression in endothelial and human cardiac fibroblast cells

Human cardiac ventricular tissues were dissociated using collagenase and the single-cell suspensions were washed, plated and cultured in DMEM supplemented with 10% FCS. HLIVECs were harvested from the human umbilical cords and cultured in EGM2. Cells passages 3-5 were used in the experiments.

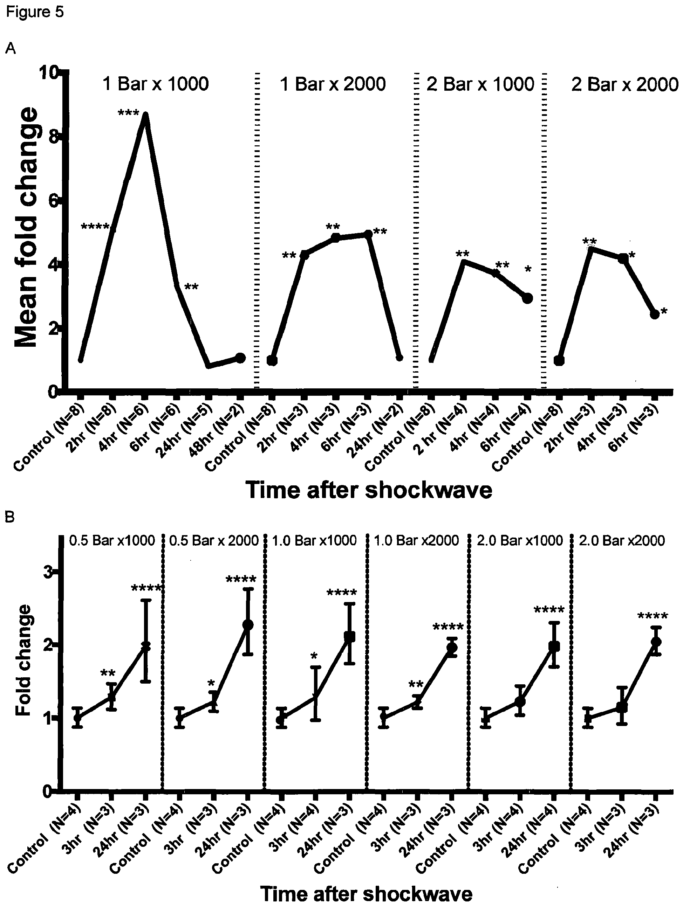

The human cardiac fibroblasts and HUVECs were subjected to shockwave treatment as described in Example 1 and upon reaching the time points, the media was removed and the cells lysed using Trizol. SDF1 gene expression was measured at various time points after shockwave treatment in accordance with the method described in Example 2. The results are shown in Figure 5.

In human cardiac fibroblasts SDF1 gene expression continued to increase at the 24-hour time point whereas SDF1 gene expression reached maximum expression between 2-6 hours in HUVECs and returned to baseline levels within 24 hours of shockwave treatment. These results demonstrate that shockwave treatment induced sustained SDF1 gene expression specifically in cardiac fibroblasts.

### Example 4: Effect of shockwave treatment on gene expression in human cardiac fibroblasts

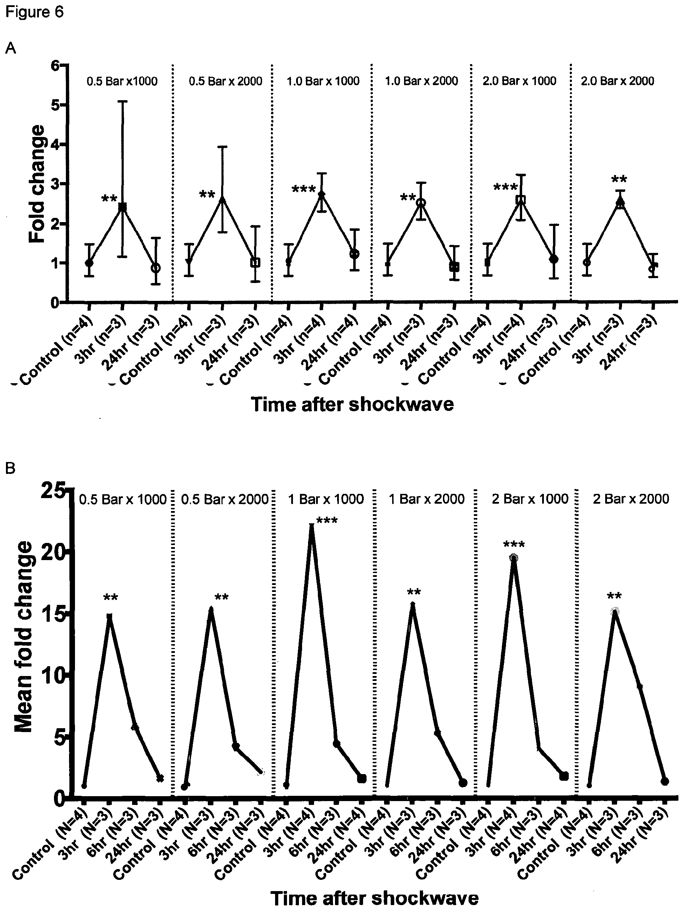

Human cardiac fibroblasts were obtained according to the method described in Example 3 and subjected to shockwave treatment as described in Example 1. VEGFA and MCP1 gene expression was measured at various time points after shockwave treatment in accordance with the method described in Example 2. The results are shown in Figure 6 and show that, in contrast with SDF1, VEGFA and MCP1 gene expression rapidly increased by the 3 hour time point and returned to baseline levels by the 24 hour time point. This data demonstrates that SDF1 expression in fibroblasts lags behind the SDF1 expression of the endothelial cells by 24hr, creating a temporal and spatial gradient from intravascular to cardiac tissue.

### Example 5: Effect of shockwave treatment on AKT phosphorylation in rat cardiomyocytes

Rat cardiomyocytes were cultured in standard conditions and subjected to treatment with: shockwaves (1000 pulses at 1 bar); exogenous SDF; or a PI3 kinase inhibitor (LY294002). AKT phosphorylation was measured at various time points using Western Blot normalised using pan-AKT and COX IV as a loading control.

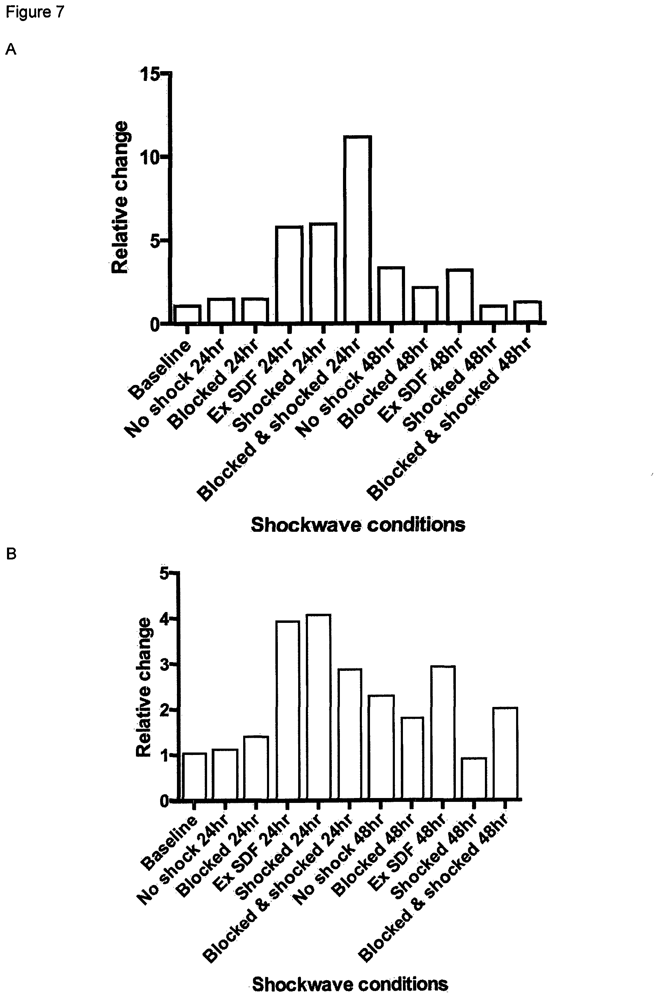

The results are show in in Figure 7 and show that AKT phosphorylation is increased in shockwave treated cells. However, the effect was not blocked by PI3 kinase inhibitor (LY294002) indicating that the phosphorylation of AKT by shockwave is independent of Phosphatidylinositol 3-kinase, an activator of the AKT pathway.

### Example 6: Effect of shockwave treatment on SDF-1 gene expression in rat cardiomyocytes

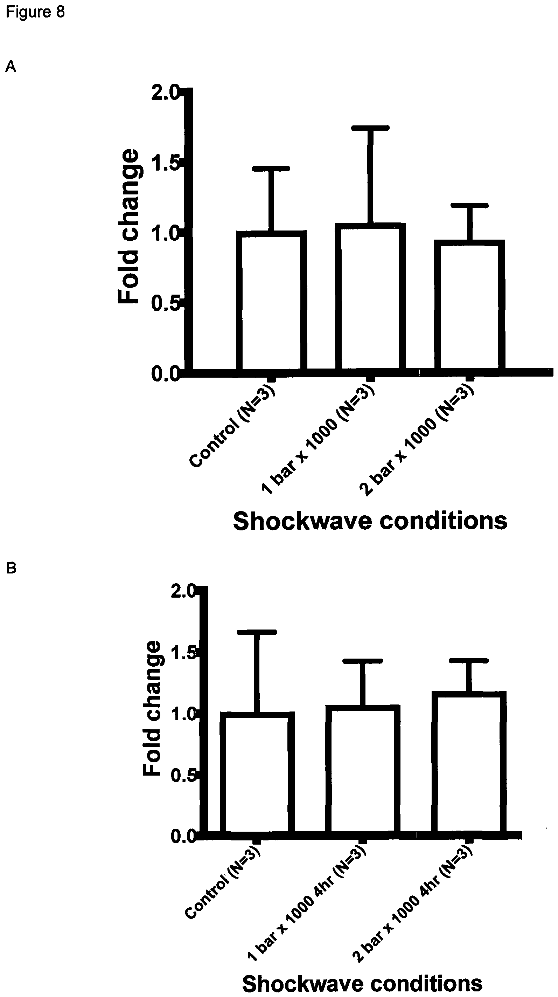

Rat cardiomyocytes were cultured according to the method in Example 1 and subjected to treatment with shockwaves (1000 pulses at 1 bar or 2 bar). SDF1 and VEGFA gene expression was measured 4 hours after treatment as described in Example 2. The results are shown in Figure 8 and demonstrate that there is no statistically significance difference between the untreated controls and the shockwave conditions for both SDF1 and VEGFA gene expression. This indicates that the anti-apoptotic effect of shockwaves on cardiomyocytes is independent of anti-apoptotic factors such as SDF1 and VEGFA.

### Example 7: Effects of shockwave and DPP-4 inhibitor (DPP4i) treatments on rat hearts

Male Lewis rats (250-275g) were subjected to the following treatments for four days: (1) no treatment; (2) extracorporeal cardiac shockwave treatment on day 2 of 4 and daily water oral gavages; (3) extracorporeal cardiac shockwave treatment on day 2 of 4 and daily DPP4i oral gavages; and (4) daily DPP4i oral gavages.

Shockwave treatments (0.25mJ/mm² x 1000 pulses at 4Hz using a Storz Medical DUOLITH^{®} SD1 device) were administered under general anaesthesia (1.5-2% isoflurane in 100% O₂). The shockwaves were administered continuously and targeted to the heart, which was located using finger palpation and echocardiography coupled with ultrasound gel.

DPP4 inhibitor (Linagliptin, 3mg per rat delivered as a 1mg/ml solution) or water was administered directly into the stomach via a gavage needle.

The animals were culled and the hearts were explanted 4 days after the onset of the experiment. Blood was washed off the hearts with phosphate buffered saline, fixed in 4% paraformaldehyde overnight, cryoprotected in 30% sucrose overnight and then embedded in OCT for cryosectioning using a Cryostat.

Immunohistochemistry was performed on sections blocked with 1% BSA in Tris-buffered saline + Tween using rabbit anti-rat SDF1 primary antibodies and goat anti-rabbit HRP-conjugated (horse radish peroxidase-conjugated) secondary antibodies. The tissue sections were counterstained using hematoxylin and 3,3'-diaminobenzidine (DAB) substrate was used for chromogenic detection of HRP. DAB results in a brown insoluble product in the presence of HRP. Sections were dehydrated using ethanol series, mounted using DPX-new and Xylene replacement as the solvent. Bright-field light microscopy was used to visualise the sections.

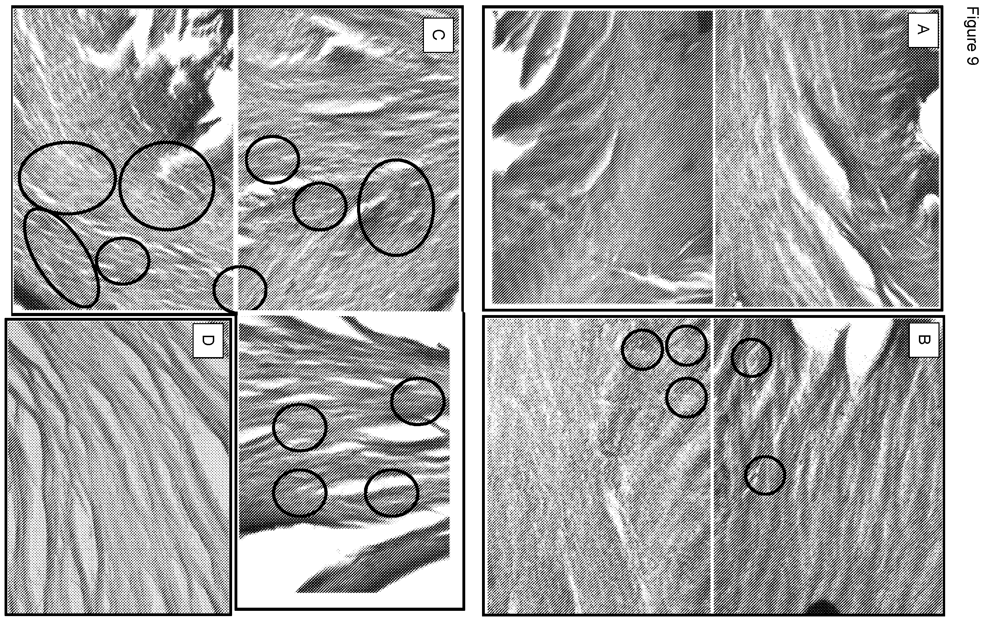

The results are shown in Figure 9, wherein treatments 1-4 are shown in Figures 9A-D, respectively. For ease of reference, deposits of the brown insoluble reaction product, which are evidence of the presence of SDF-1, are circled. It was observed that the SDF-1 was induced by extracorporeal shockwave therapy (see Figures 9B and 9C); no brown deposits were observed in heart tissue from untreated rats (Figure 9A) or rats treated only with DPP4i (Figure 9D). Moreover, the rats treated with DPP4i showed a significant increase in SDF-1 relative to rats treated only with shockwaves or DPP4i. These results indicate that the combination of shockwave and DPP4i treatment results in a more than additive effect on the presence of SDF-1 in cardiac tissue.

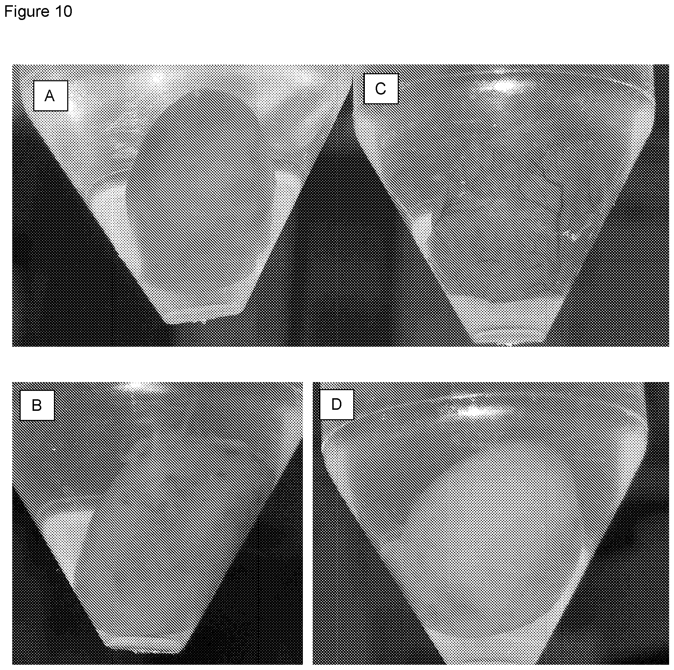

Figure 10 shows appearance of rat hearts after overnight incubation with 4% paraformaldehyde: A) untreated normal control, B) shockwave only; C) shockwave and DPP4i, and D) DPP4i only. It was observed that hearts from rats treated with shockwave and DPP4i have very prominent blood vessels compared to hearts from rats treated only with shockwaves. Hearts from untreated rats and rats treated only with DPP4i are very similar. It was concluded that DPP4i enhances the angiogenic process induced by shockwave and DPP4i on its own has a neutral effect.

## Claims

1. A dipeptidyl peptidase-4 (DPP-4) inhibitor, or a pharmaceutical composition containing said inhibitor, for use in:
(a) treating or preventing heart failure; or
(b) treating cardiac ischaemic reperfusion injury,
in a subject, wherein said subject has been administered extracorporeal cardiac shockwave therapy and wherein said inhibitor is for administration prior to, simultaneously with, and/or after administration of said shockwave therapy.

2. The DPP-4 inhibitor or composition for use according to claim 1, wherein said inhibitor or composition is provided as a combined preparation with a pharmacological agent suitable for mobilising stem cells for separate, simultaneous or sequential use or administration.

3. The DPP-4 inhibitor or composition for use according to claim 2, wherein said pharmacological agent suitable for mobilising stem cells is parathyroid hormone or a fragment thereof, optionally wherein said parathyroid hormone fragment comprises teriparatide.

4. The DPP-4 inhibitor or composition for use according to any one of claims 1 to 3, wherein said inhibitor or composition is for administration prior to said shockwave therapy administration and continues throughout the course of the shockwave therapy.

5. The DPP-4 inhibitor or composition for use according to any one of claims 2 to 4, wherein the pharmacological agent suitable for mobilising stem cells is for administration at least 8 hours before the shockwave therapy.

6. The DPP-4 inhibitor or composition for use according to claim 1, wherein said inhibitor is provided as a combined preparation with stem cells for separate, simultaneous or sequential use or administration, optionally wherein said stem cells are derived from or comprise bone marrow-derived mononuclear cells.

7. The DPP-4 inhibitor or composition for use according to claim 5 or 6, wherein the stem cells are for administration at least 8 hours before the shockwave therapy.

8. The DPP-4 inhibitor or composition for use according to any one of claims 1 to 7, wherein the DPP-4 inhibitor is Sitagliptin, Linagliptin, Vildagliptin, Gemigliptin, Anagliptin, Teneligliptin, Trelagliptin, Dutogliptin, Omarigliptin, Lupeol or a combination thereof.

9. The DPP-4 inhibitor or composition for use according to any one of claims 1 to 8, wherein said heart failure is chronic heart failure or post-infarction heart failure.

10. The DPP-4 inhibitor or composition for use according to any one of claims 1 to 8, wherein said shockwave therapy comprises administration of shockwave pulses with energy of about 0.05-0.25 mJ/mm² and/or said shockwave therapy comprises administration of at least about 500 shockwave pulses, optionally wherein said shockwave therapy comprises administration of a dose of about 500-2000 shockwave pulses.

11. The DPP-4 inhibitor or composition for use according to any one of claims 1 to 10, wherein said shockwave therapy comprises administration of shockwave pulses during the isovolumic contraction and/or isovolumic relaxation periods of the cardiac cycle and/or wherein the focal point of the shockwave pulses is targeted to a specific region of the heart, optionally wherein the region of the heart to be targeted is determined by echocardiography (Echo) visualisation and/or electrocardiogram (ECG) pattern recognition or magnetic resonance imaging.

12. The DPP-4 inhibitor or composition for use according to claim 11,
wherein specific region of the heart to be targeted comprises scar tissue.

13. The DPP-4 inhibitor or composition for use according to any one of claims 1 to 12, wherein the dose of shockwave pulses is for administration over more than one session.

14. A kit or product containing:
(i) a DPP-4 inhibitor or composition comprising said inhibitor; and
(ii) a pharmacological agent for mobilising stem cells, such as parathyroid hormone or fragment thereof, or a pharmaceutical composition comprising said pharmacological agent and at least one pharmaceutically acceptable carrier, diluent or excipient; and optionally
(iii) an additional agent, e.g. an agent for treatment of heart failure or glucose, or a pharmaceutical composition comprising said additional agent and at least one pharmaceutically acceptable carrier, diluent or excipient,
wherein (i), (ii) and optionally (iii) are provided as a combined preparation for simultaneous, sequential or separate use in:
(a) treating or preventing heart failure; or
(b) treating cardiac ischaemic reperfusion injury,
in a subject, wherein said subject has been administered extracorporeal cardiac shockwave therapy and wherein said inhibitor and/or pharmacological agent for mobilising stem cells is for administration prior to, simultaneously with, and/or after administration of said shockwave therapy.

15. The kit or product of claim 14, wherein said a DPP-4 inhibitor, pharmacological agent for mobilising stem cells and/or shockwave therapy is as defined in any of the aforementioned claims.

## Patentansprüche

1. Inhibitor der Dipeptidyl-Peptidase-4 (DPP-4) oder eine pharmazeutische Zusammensetzung, die diesen Inhibitor enthält, zur Verwendung bei der:
(a) Behandlung oder Vorbeugung von Herzinsuffizienz; oder
(b) Behandlung von kardialen ischämischen Reperfusionsschäden,
bei einem Subjekt, wobei dem Subjekt eine extrakorporale kardiale Stoßwellentherapie verabreicht wurde und wobei der Inhibitor zur Verabreichung vor, gleichzeitig mit, und/oder nach Verabreichung der Stoßwellentherapie bestimmt ist.

2. DPP-4-Inhibitor oder Zusammensetzung zur Verwendung nach Anspruch 1, wobei der Inhibitor oder die Zusammensetzung als Kombinationspräparat mit einem zum Mobilisieren von Stammzellen geeigneten pharmakologischen Wirkstoff zur separaten, gleichzeitigen oder aufeinanderfolgenden Verwendung oder Verabreichung bereitgestellt wird.

3. DPP-4-Inhibitor oder Zusammensetzung zur Verwendung nach Anspruch 2, wobei der zum Mobilisieren von Stammzellen geeignete pharmakologische Wirkstoff Parathormon oder ein Fragment davon ist, wobei das Parathormonfragment optional Teriparatid umfasst.

4. DPP-4-Inhibitor oder Zusammensetzung zur Verwendung nach einem der Ansprüche 1 bis 3, wobei der Inhibitor oder die Zusammensetzung zur Verabreichung vor der Stoßwellentherapie bestimmt ist und während der gesamten Stoßwellentherapie fortgesetzt wird.

5. DPP-4-Inhibitor oder Zusammensetzung zur Verwendung nach einem der Ansprüche 2 bis 4, wobei der zur Mobilisierung von Stammzellen geeignete pharmakologische Werkstoff zur Verabreichung mindestens 8 Stunden vor der Stoßwellentherapie bestimmt ist.

6. DPP-4-Inhibitor oder Zusammensetzung zur Verwendung nach Anspruch 1, wobei der Inhibitor als ein Kombinationspräparat mit Stammzellen zur getrennten, gleichzeitigen oder aufeinanderfolgenden Verwendung oder Verabreichung bereitgestellt wird, wobei die Stammzellen optional aus Knochenmark stammenden mononukleären Zellen stammen oder solche umfassen.

7. DPP-4-Inhibitor oder Zusammensetzung zur Verwendung nach Anspruch 5 oder 6, wobei die Stammzellen zur Verabreichung mindestens 8 Stunden vor der Stoßwellentherapie bestimmt sind.

8. DPP-4-Inhibitor oder Zusammensetzung zur Verwendung nach einem der Ansprüche 1 bis 7, wobei der DPP-4-Inhibitor Sitagliptin, Linagliptin, Vildagliptin, Gemigliptin, Anagliptin, Teneligliptin, Trelagliptin, Dutogliptin, Omarigliptin, Lupeol oder eine Kombination davon ist.

9. DPP-4-Inhibitor oder Zusammensetzung zur Verwendung nach einem der Ansprüche 1 bis 8, wobei es sich bei der Herzinsuffizienz um eine chronische Herzinsuffizienz oder eine Herzinfarktinsuffizienz handelt.

10. DPP-4-Inhibitor oder Zusammensetzung zur Verwendung nach einem der Ansprüche 1 bis 8, wobei die Stoßwellentherapie die Verabreichung von Stoßwellenimpulsen mit einer Energie von etwa 0,05 bis 0,25 mJ/mm² umfasst und/oder die Stoßwellentherapie die Verabreichung von mindestens etwa 500 Stoßwellenimpulsen umfasst, wobei die Stoßwellentherapie optional die Verabreichung einer Dosis von etwa 500-2000 Stoßwellenimpulsen umfasst.

11. DPP-4-Inhibitor oder Zusammensetzung zur Verwendung nach einem der Ansprüche 1 bis 10, wobei die Stoßwellentherapie die Verabreichung von Stoßwellenimpulsen während der isovolumischen Kontraktion und/oder isovolumischer Entspannungsphasen des Herzzyklus umfasst und/oder wobei der Brennpunkt der Stoßwellenimpulse auf einen bestimmten Bereich des Herzens abzielt, wobei optional der anzuvisierende Bereich des Herzens durch Echokardiographie-Visualisierung (Echo-Visualisierung) und/oder Elektrokardiogramm-Mustererkennung (EKG-Mustererkennung) oder Magnetresonanztomographie bestimmt wird.

12. DPP-4-Inhibitor oder Zusammensetzung zur Verwendung nach Anspruch 11, wobei der spezifische Zielbereich des Herzens Narbengewebe umfasst.

13. DPP-4-Inhibitor oder Zusammensetzung zur Verwendung nach einem der Ansprüche 1 bis 12, wobei die Dosis der Stoßwellenimpulse zur Verabreichung über mehr als eine Sitzung bestimmt ist.

14. Kit oder Produkt, das Folgendes enthält:
(i) einen DPP-4-Inhibitor oder eine Zusammensetzung, die diesen Inhibitor umfasst; und
(ii) einen pharmakologischen Wirkstoff zum Mobilisieren von Stammzellen, wie Parathormon oder ein Fragment davon, oder eine pharmazeutische Zusammensetzung, die diesen pharmakologischen Wirkstoff und mindestens einen pharmazeutisch akzeptablen Träger, Verdünnungsmittel oder Hilfsstoff umfasst; und optional
(iii) einen zusätzlichen Wirkstoff, z. B. einen Wirkstoff zur Behandlung von Herzinsuffizienz oder Glucose, oder eine pharmazeutische Zusammensetzung, die den zusätzlichen Wirkstoff und mindestens einen pharmazeutisch verträglichen Träger, ein Verdünnungsmittel oder einen Hilfsstoff umfasst,
wobei (i), (ii) und optional (iii), als Kombinationspräparat bereitgestellt werden zur gleichzeitigen, aufeinanderfolgenden oder getrennten Verwendung bei der:
(a) Behandlung oder Vorbeugung von Herzinsuffizienz; oder
(b) Behandlung von kardialen ischämischen Reperfusionsschäden,
bei einem Subjekt, wobei dem Subjekt eine extrakorporale kardiale Stoßwellentherapie verabreicht wurde und wobei der Inhibitor und/oder pharmakologische Wirkstoff zum Mobilisieren von Stammzellen zur Verabreichung vor, gleichzeitig mit, und/oder nach Verabreichung der Stoßwellentherapie bestimmt ist.

15. Kit oder Produkt nach Anspruch 14, wobei der eine DPP-4-Inhibitor, pharmakologische Wirkstoff zum Mobilisieren von Stammzellen und/oder die Stoßwellentherapie wie in einem der oben stehenden Ansprüche definiert ist.

## Revendications

1. Inhibiteur de la dipeptidylpeptidase-4 (DPP-4), ou composition pharmaceutique contenant ledit inhibiteur, pour une utilisation dans :
(a) le traitement ou la prévention de l'insuffisance cardiaque ; ou
(b) le traitement de lésions de reperfusion ischémique cardiaque,
chez un sujet, dans lequel ledit sujet a reçu une thérapie par ondes de choc cardiaques extracorporelles et dans lequel ledit inhibiteur est destiné à être administré avant, en même temps que et/ou après l'administration de ladite thérapie par ondes de choc.

2. Inhibiteur de DPP-4 ou composition pour une utilisation selon la revendication 1, dans lequel ledit inhibiteur est fourni, ou dans laquelle ladite composition est fournie, sous la forme d'une préparation combinée avec un agent pharmacologique approprié pour mobiliser des cellules souches pour une utilisation ou une administration séparée, simultanée ou séquentielle.

3. Inhibiteur de DPP-4 ou composition pour une utilisation selon la revendication 2, dans lequel ledit agent pharmacologique approprié pour mobiliser des cellules souches est l'hormone parathyroïdienne ou un fragment de celle-ci, facultativement dans lequel/laquelle ledit fragment d'hormone parathyroïdienne comprend du tériparatide.

4. Inhibiteur de DPP-4 ou composition pour une utilisation selon l'une quelconque des revendications 1 à 3, dans lequel ledit inhibiteur est destiné à être administré, ou dans laquelle ladite composition est destinée à être administrée, avant ladite administration de thérapie par ondes de choc, et se poursuit tout au long de la thérapie par ondes de choc.

5. Inhibiteur de DPP-4 ou composition pour une utilisation selon l'une quelconque des revendications 2 à 4, dans lequel/laquelle l'agent pharmacologique approprié pour mobiliser des cellules souches est destiné à être administré au moins 8 heures avant la thérapie par ondes de choc.

6. Inhibiteur de DPP-4 ou composition pour une utilisation selon la revendication 1, dans lequel/laquelle ledit inhibiteur est fourni sous la forme d'une préparation combinée avec des cellules souches pour une utilisation ou une administration séparée, simultanée ou séquentielle, facultativement dans lequel/laquelle lesdites cellules souches sont dérivées de cellules mononucléaires, ou comprennent celles-ci, dérivées de la moelle osseuse.

7. Inhibiteur de DPP-4 ou composition pour une utilisation selon la revendication 5 ou 6, dans lequel/laquelle les cellules souches sont destinées à être administrées au moins 8 heures avant la thérapie par ondes de choc.

8. Inhibiteur de DPP-4 ou composition pour une utilisation selon l'une quelconque des revendications 1 à 7, dans lequel/laquelle l'inhibiteur de DPP-4 est la sitagliptine, la linagliptine, la vildagliptine, la gemigliptine, l'anagliptine, la ténéligliptine, la trélagliptine, la dutogliptine, l'omarigliptine, le lupéol ou une combinaison de ceux-ci.

9. Inhibiteur de DPP-4 ou composition pour une utilisation selon l'une quelconque des revendications 1 à 8, dans lequel/laquelle ladite insuffisance cardiaque est une insuffisance cardiaque chronique ou une insuffisance cardiaque post-infarctus.

10. Inhibiteur de DPP-4 ou composition pour une utilisation selon l'une quelconque des revendications 1 à 8, dans lequel/laquelle ladite thérapie par ondes de choc comprend une administration d'impulsions d'ondes de choc avec une énergie d'environ 0,05-0,25 mJ/mm² et/ou ladite thérapie par ondes de choc comprend une administration d'au moins environ 500 impulsions d'ondes de choc, facultativement dans lequel/laquelle ladite thérapie par ondes de choc comprend l'administration d'une dose d'environ 500-2000 impulsions d'ondes de choc.

11. Inhibiteur de DPP-4 ou composition pour une utilisation selon l'une quelconque des revendications 1 à 10, dans lequel/laquelle ladite thérapie par ondes de choc comprend une administration d'impulsions d'ondes de choc pendant les périodes de contraction isovolumique et/ou de relaxation isovolumique du cycle cardiaque et/ou dans lequel/laquelle le point focal des impulsions d'ondes de choc est ciblé sur une région spécifique du coeur, facultativement dans lequel/laquelle la région du coeur à cibler est déterminée par visualisation par échocardiographie (Echo) et/ou reconnaissance de motif d'électrocardiogramme (ECG) ou imagerie par résonance magnétique.

12. Inhibiteur ou composition de DPP-4 pour une utilisation selon la revendication 11, dans lequel/laquelle la région spécifique du coeur à cibler comprend du tissu cicatriciel.

13. Inhibiteur de DPP-4 ou composition pour une utilisation selon l'une quelconque des revendications 1 à 12, dans lequel/laquelle la dose d'impulsions d'ondes de choc est destinée à être administrée sur plus d'une séance.

14. Kit ou produit, contenant
(i) un inhibiteur de DPP-4 ou une composition comprenant ledit inhibiteur ; et
(ii) un agent pharmacologique pour mobiliser des cellules souches, comme l'hormone parathyroïdienne ou un fragment de celle-ci, ou une composition pharmaceutique comprenant ledit agent pharmacologique et au moins un support, diluant ou excipient pharmaceutiquement acceptable ; et facultativement
(iii) un agent supplémentaire, par exemple un agent pour le traitement de l'insuffisance cardiaque ou du glucose, ou une composition pharmaceutique comprenant ledit agent supplémentaire et au moins un support, diluant ou excipient pharmaceutiquement acceptable,
dans lequel (i), (ii) et facultativement (iii) sont fournis sous la forme d'une préparation combinée pour une utilisation simultanée, séquentielle ou séparée dans :
(a) le traitement ou la prévention de l'insuffisance cardiaque ; ou
(b) le traitement de lésions de reperfusion ischémique cardiaque,
chez un sujet, dans lequel ledit sujet a reçu une thérapie par ondes de choc cardiaques extracorporelles et dans lequel ledit inhibiteur et/ou ledit agent thérapeutique pour mobiliser des cellules souches est destiné à être administré avant, en même temps que et/ou après l'administration de ladite thérapie par ondes de choc.

15. Kit ou produit selon la revendication 14, dans lequel ledit inhibiteur de DPP-4, ledit agent pharmacologique pour mobiliser des cellules souches et/ou ladite thérapie par ondes de choc sont tels que définis dans l'une quelconque des revendications mentionnées ci-dessus.
